# EUROPEAN PATENT APPLICATION

(11) **EP 0 549 291 A1**
(43) Date of publication of application: **30.06.1993**
(21) Application number: 92311626.3
(22) Date of filing: 17.12.1992
(51) Int. Cl.: C07D 487/04, A61K 31/505

(54) **Thymidylate synthase inhibitors**

(30) Priority: 27.12.1991 JP 347195/91; 08.10.1992 JP 270252/92
(71) Applicant: TAKEDA CHEMICAL INDUSTRIES, LTD., Chuo-ku, Osaka 541 (JP)
(72) Inventor: Akimoto, Hiroshi, Kobe, Hyogo 658 (JP); Aso, Kazuyoshi, Osaka 552 (JP)
(74) Representative: Horton, Sophie Emma (GB)

(57) **Abstract**

Novel compounds of the general formula: 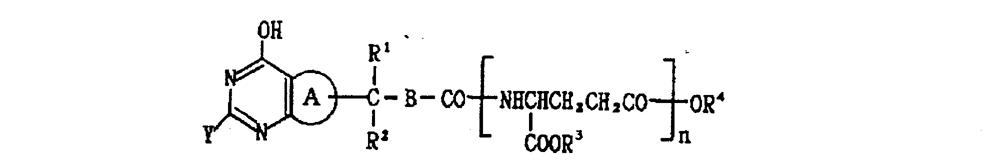
wherein a ring A represents a pyrrole or pyrroline ring which may be substituted; B means a divalent 5- or 6-membered homo- or hetero-cyclic group which may be substituted; Y means a hydrogen atom, a halogen atom, or a group bonded through a carbon, nitrogen or sulfur atom; R¹ and R² each means a hydrogen atom or a lower hydrocarbon group which may be substituted; -COOR³ and -COOR⁴ each means a carboxyl group which may be esterified; n means a whole number of 1 through 6, or a salt thereof, possessing suprisingly high inhibitory activity against thymidylate synthase, and their production and use.

## Description

The present invention relates to thymidylate synthase inhibitors, novel condensed pyrimidine derivatives as their active component, production process therefor, and intermediate compounds.

Folic acid and its related compounds, as carriers of one-carbon units derived from formic acid, formaldehyde, etc. in the living body, act as coenzymes in the various enzymatic reactions such as nucleic acid biosynthesis, amino acid and peptide metabolisms, methane generation, and other biological systems. Particularly in nucleic acid biosynthesis, they are indispensable for the metabolism or transfer reactions of one-carbon units in two pathways, namely the purine synthesis and pyrimidine synthesis pathways. Usually in order that folic acid may exhibit its biological activities, it must be reduced in two steps to the active coenzyme form. As drugs which are intimately bound to the enzyme controlling the second reduction step (dihydrofolate reductase, DHFR) to thereby inhibit reduction of dihydrofolic acid to tetrahydrofolic acid, there are known amethopterin (methotrexate: MTX) and its related compounds. Since these drugs impair DNA synthesis and, hence, cause cell death, they are clinically valued much as therapeutic agents for leukemia and other diseases. Furthermore, with the amazing progress of folic acid-related research in the field of biochemistry and particularly in association with cancer research, there have been reported several new DHFR inhibitors such as 10-deazaaminopterin derivatives [10-ethyl-10-deazaaminopterin: 10-EDAM] [NCI Monograph 5, 127 (1987)] and aminopteroyl-ornithine derivatives [N(α)-(4-amino-4-deoxypteroyl)-N(δ)-hemiphthaloyl-L-ornithine : PT523] [USP4,767,761] and antagonistic inhibitors aimed at enzymes other than DHFR, such as 5-deazatetrahydroaminopterin antitumoral agents whose principal mechanism of action is inhibition of glycinamide ribonucleotide transformylase [5,10-dideaza-5,6,7,8-tetrahydroaminopterin: DDATHF] [Journal of Medicinal Chemistry 28, 914 (1985)] and quinazoline antitumoral agents whose principal mechanism of action is inhibition of thymidylate synthase [2-desamino-2-methyl-10-propargyl-5,8-dideazafolate: DMPDDF] [British Journal of Cancer 58, 241 (1988)]. However, all of these compounds belong to the category of heterocyclic compounds whose skeletal structure is a fused ring structure between a 6-membered ring and another 6-membered ring (the 6-6 fused ring). Meanwhile, folate antagonists having the skeletal fused ring structure formed between a 6-membered ring and a 5-membered ring (the 6-5 fused ring), instead of two 6-membered rings, namely the pyrrolo[2,3-d]pyrimidine ring, are also reported to have high antitumoral activity (USP4,997,838, EP-A-400,562, EP-A-402,903, EP-A-418,924, EP-A-431,953, EP-A-434,426, EP-A-438,261, USP4,996,206, USP4,895,946, EP-A-492,316 and USP Application SN 07/926170.

The most pressing demand in the area of cancer therapy today is creation of drugs which would display highly selective toxicity against cancer cells and high therapeutic efficacy through new mechanisms of action. MTX whose principal mechanism of action is inhibition of dihydrofolate reductase is clinically in common use today but has not achieved dramatic therapeutic results because of fair toxicity and little effect against solid cancer. Furthermore, acquired resistance to MTX is also presenting a serious problem. Among the mechanisms of resistance to MTX are elevation of DHFR level, decreased drug transporting system of the cellular membrane and depression of folylpolyglutamate synthase (FPGS) level. It is foreseen that by overcoming one or more of these resistance factors we may develop drugs that may exhibit excellent therapeutic effects against MTX-resistant tumors.

The inventors of the present invention explored in earnest for a solution to the above problems and found that certain condensed pyrimidine derivatives which are different in chemical structure from the compounds described in USP4,997,838, EP-A-400,562, EP-A-418,924, EP-A-431,953, EP-A-434,426, EP-A-438,261, EP-A-492,316, USP4,996,206 and JP Application 202,042/1991 are possessed of surprisingly high inhibitory activity against thymidylate synthetase with which folic acid and its congeners are associated, display highly selective cytotoxicity against a variety of tumor cells (especially human lung cancer cells) and exhibit excellent therapeutic efficacy even overcoming said MTX resistance. The present invention has been developed on the basis of the above findings.

Thus, the present invention is directed to
(1) a novel compound of the general formula: 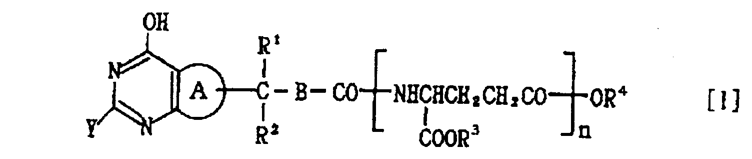 wherein a ring A represents a pyrrole or pyrroline ring which may be substituted; B means a divalent 5- or 6-membered homo- or hetero-cyclic group which may be substituted; Y means a hydrogen atom, a halogen atom, or a group bonded through a carbon, nitrogen or sulfur atom; R¹ and R² are the same or different and each means a hydrogen atom or a lower hydrocarbon group which may be substituted; -COOR³ and -COOR⁴ are the same or different and each means a carboxyl group which may be esterified; n means a whole number of 1 through 6; R³ may be different over n occurrences, or a salt thereof;
(2) a compound of the following general formula: 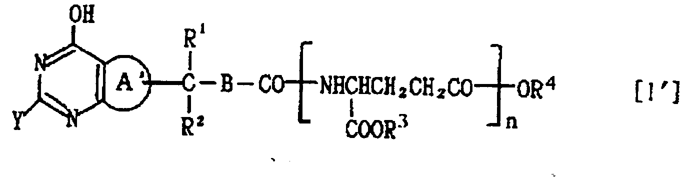 wherein a ring A' represents an N-substituted pyrrole or pyrroline ring; and the other symbols have the same meanings as defined hereinbefore, or a salt thereof;
(3) N-[4-(2-amino-4-hydroxy-7H-pyrrolo[2,3-d]pyrimidin-5-yl)methylbenzoyl]-L-glutamic acid or a salt or ester thereof;
(4) a process for producing the compound [I] or a salt thererof, characterized by reacting a compound of the general formula: 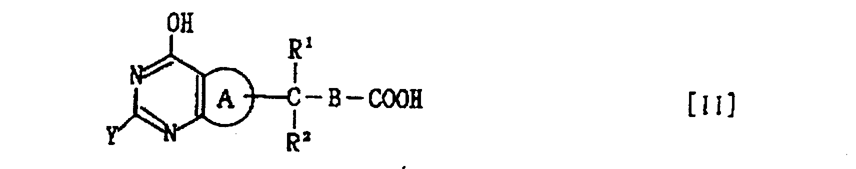 wherein the symbols have the same meanings as defined hereinbefore, or a reactive derivative of the carboxyl group thereof with a compound of the general formula: 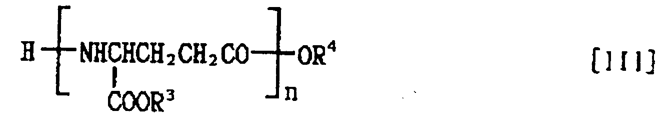 wherein the symbols have the same meanings as defined hereinbefore;
(5) a compound [II], or a salt or ester thereof,
(6) a thymidylate synthase inhibitor composition which contains the compound [I] or a pharmaceutically acceptable salt, and so on.

Referring to the above formulas, compounds [I], [I'] and [II] may each exist as an equilibrium mixture of tautomers. The following partial structural formula shows the moiety responsible for tautomerism and the state of equilibrium between tautomers. 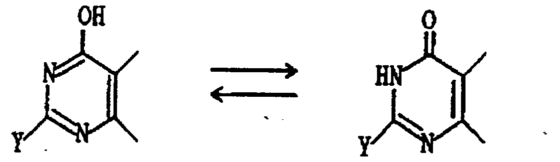
For convenience in designation, the hydroxy form is indicated and the corresponding nomenclature is used throughout this specification. In each case, however, it is intended that the tautomeric oxo form is also included.

Furthermore, compounds [I], [I'], [II] and [III] may have asymmetric centers but all that is necessary is that the absolute configuration of the partial structure of [I], [I'] and [III]: 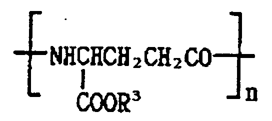
is S(L) and the absolute configuration of any other center of asymmetry may be any of S, R and RS. There exist, in such cases, diastereomers but they can be easily fractionated by the conventional purification procedure. All the diastereomers that can be fractionated in this manner fall within the scope of the present invention.

Referring to the general formula presented above, the pyrrole or pyrroline ring which may be substituted, which is represented by a ring A, may have 1 or 2 substituents in substitutable positions on the nitrogen and/or carbon atoms. Among such substituents may be included, for example, C₁₋₄ alkyl (e.g. methyl, ethyl, propyl, isopropyl), C₂₋₄ alkenyl (e.g. vinyl, 1-methylvinyl , 1-propenyl, allyl, allenyl), C₂₋₄ alkynyl (e.g. ethynyl, 1-propynyl, 2-propynyl), C₃₋₆ cycloalkyl (e.g. cyclopropyl), halogen (e.g. fluorine, chlorine, bromine, iodine), C₁₋₄ alkanoyl (e.g. formyl, acetyl, propionyl, butyryl, isobutyryl), benzoyl, substituted benzoyl (e.g. halobenzoyl such as p-chlorobenzoyl; mono-, di- or tri-C₁₋₄ alkoxybenzoyl such as p-methoxybenzoyl, 3,4,5-trimethoxybenzoyl, etc.), cyano, carboxy, carbamoyl, nitro, hydroxy, hydroxy-C₁₋₄ alkyl (e.g. hydroxymethyl, hydroxyethyl), C₁₋₄alkoxy-C₁₋₄alkyl (e.g. methoxymethyl, ethoxymethyl, methoxyethyl, ethoxyethyl), C₁₋₄ alkoxy (e.g. methoxy, ethoxy, propoxy), mercapto, C₁₋₄ alkylthio (e.g. methylthio, ethylthio, propylthio), amino, mono- or di-C₁₋₄ alkylamino (e.g. methylamino, ethylamino, dimethylamino, diethylamino), C₁₋₄ alkanoylamino (e.g. formamido, acetamide), phenyl, substituted phenyl (e.g. halophenyl such-as p-chlorophenyl etc., mono-, di- or tri-C₁₋₄ alkoxyphenyl such as p-methoxyphenyl, 3,4,5-trimethoxyphenyl), benzyl, substituted benzyl (e.g. halobenzyl such as p-chlorobenzyl, etc., C₁₋₄ alkoxybenzyl such as p-methoxybenzyl etc.; diphenylmethyl) and so on. The preferred, among the above, are C₁₋₄ alkyl, C₂₋₄alkenyl, C₂₋₄ alkynyl, C₁₋₄ alkanoyl, benzoyl, substituted benzoyl, hydroxy-C₁₋₄ alkyl, C₁₋₄ alkoxy-C₁₋₄ alkyl, benzyl and substituted benzyl groups.

Regarding the bonding of A to 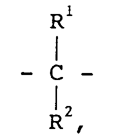
the ring may be bound to 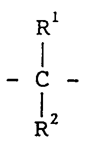 in any available position on the nitrogen or carbon atom of the pyrrole or pyrroline ring but is preferably bound in the β-position of the pyrrole or pyrroline ring.

B means a divalent 5- or 6-membered homo- or hetero-cyclic group which may be substituted and the two bonds are preferably not in the vicinal positions of the ring. The homocyclic group B may for example be a divalent 5- or 6-membered cyclic hydrocarbon group. Such a hydrocarbon group may for example be a 5- or 6-membered alicyclic hydrocarbon group (e.g. cyclopentylene, cyclohexylene) or phenylene. The heterocyclic group B is a divalent 5- or 6-membered heterocyclic group containing 1 to 3 hetero-atoms (e.g. N, O, S) as ring-constituent members. Among such 5-membered cyclic groups, represented by B, are 1,3- or 3,5-cyclopentadien-1,3-ylene, cyclopenten-(1,3-, 1,4- or 3,5-)ylene, cyclopentan-1,3-ylene, thiophen-(2,4-, 2,5-or 3,4-)ylene, furan-(2,4-, 2,5- or 3,4-)ylene, pyrrol-(1,3-, 2,4-, 2,5- or 3,4-)ylene, thiazol-(2,4- or 2,5-)ylene, imidazol-(1,4-, 2,4- or 2,5-)ylene, thiadiazol-2,5-ylene, etc. and the corresponding partially reduced forms (the unsaturation has been partially reduced) or completely reduced forms (the unsaturation has been completely reduced). Among the 6-membered cyclic groups which can be used are phenyl-(1,3- or 1,4-)ylene, cyclohexan-(1,3- or 1,4-)ylene, cyclohexen-(1,3-, 1,4-, 1,5-, 3,5- or 3,6-)ylene, 1,3-cyclohexadien-(1,3-, 1,4-, 1,5-, 2,4-, 2,5- or 2,6-)ylene, 1,4-cyclohexadien-(1,3-, 1,4- or 1,5-)ylene, pyridin-(2,4-, 2,5-, 2,6- or 3,5-)ylene, pyran-(2,4-, 2,5-, 2,6-, 3,5-, 3,6- or 4,6-)ylene, pyrazin-(2,5- or 2,6-)ylene, pyrimidin-(3,4- or 2,5-)ylene, pyridazin-3,5-ylene, etc. and the corresponding partially reduced and completely reduced forms. Among particularly preferred examples of B are phenyl-1,4-ylene, thiophen-2,5-ylene, thiazol-2,5-ylene and pyridin-2,5-ylene. The divalent 5- or 6-membered homo- or hetero-cyclic group B may have 1 or 2 substituents in substitutable positions. As such substituents, there may be mentioned, inter alia, C₁₋₄ alkyl (e.g. methyl, ethyl, propyl, isopropyl), C₂₋₄ alkenyl (e.g. vinyl, 1-methylvinyl , 1-propenyl, allyl, allenyl), C₂₋₄ alkynyl (ethynyl, 1-propynyl, 2-propynyl), C₃₋₆ cycloalkyl (e.g cyclopropyl), halogen (e.g. chlorine, bromine, fluorine, iodine), hydroxy, C₁₋₄ alkoxy (e.g. methoxy), di-C₁₋₄ alkylamino (dimethylamino), halo-C₁₋₄alkyl (e.g. trifluoromethyl), oxo, C₁₋₄ acyl (e.g. formyl) and C₁₋₄ alkoxy-C₁₋₄ alkyl (e.g. methoxymethyl, 2-ethoxyethyl).

The halogen atom designated by the symbol Y may for example be fluorine, chlorine, bromine or iodine.

The group bonded through a carbon, nitrogen, oxygen or sulfur atom, which is also designated by Y, includes such groups as cyano,carboxy, carbamoyl, amino, nitro, hydroxy, mercapto, lower hydrocarbon groups such as C₁₋₄ alkyl (e.g. methyl, ethyl, propyl, isopropyl), C₂₋₄ alkenyl (e.g. vinyl, 1-methylvinyl, 1-propenyl, allyl, allenyl), C₂₋₄ alkynyl (e.g. ethynyl, 1-propynyl, 2-propynyl), and C₃₋₆ cycloalkyl (e.g. cyclopropyl). Aside from the above, there may also be mentioned C₆₋₁₀ aryl (e.g. phenyl, naphthyl), 5- or 6-membered heterocyclic groups containing 1 to 4 hetero-atoms such as N, S and O (e.g. pyrrolyl, imidazolyl, pyrazolyl, thenyl, furyl, thiazolyl, thiadiazolyl, oxazolyl, oxadiazolyl, pyridyl, pyranyl, pyrazinyl, pyrimidinyl, pyridazinyl, etc., the corresponding partially or completely reduced groups, dioxolanyl, piperidino, morpholino, N-methylpiperazinyl, N-ethylpiperazinyl, dioxanyl) and so on. When Y is a lower hydrocarbon group, an aryl group or a 5- or 6-membered heterocyclic group, it may have 1 to 2 substituents, such as C₁₋₄ alkyl (e.g. methyl, ethyl, propyl, isopropyl), C₂₋₄ alkenyl (e.g. vinyl, 1-methylvinyl, 1-propenyl, allyl, allenyl), C₂₋₄ alkynyl (e.g. ethynyl, 1-propynyl, 2-propynyl), C₃₋₈ cycloalkyl (e.g. cyclopropyl), halogen (e.g. fluorine), hydroxy, oxo, C₁₋₄ alkoxy (e.g. methoxy), di-C₁₋₄ alkylamino (e.g. dimethylamino, diethylamino), halo-C₁₋₄ alkyl (e.g. trifluoromethyl), C₁₋₄ acyl (e.g. formyl), hydroxy-C₁₋₄ alkyl (e.g. hydroxymethyl, 2-hydroxyethyl), C₁₋₄ alkoxy-C₁₋₄ alkyl (e.g. methoxymethyl, 2-ethoxyethyl) and so on. The group bonded through a carbon, nitrogen, oxygen or sulfur atom, represented by Y, further includes alkoxy, alkylthio, alkylcarbonylamino and alkylcarbonyloxy groups and the alkyl moieties of such groups may be those groups specifically mentioned above for the lower hydrocarbon groups represented by Y.

The group bonded through a carbon, nitrogen, oxygen or sulfur atom, represented by Y, further includes aryloxy, arylthio, aroylamino and aroyloxy groups and the aryl moieties of these groups may be phenyl, naphthyl and so on as mentioned above for the C₆₋₁₀ aryl represented by Y. Furthermore, the group bonded through a carbon, nitrogen, oxygen or sulfur atom, represented by Y, may also be selected from among heterocyclyloxy, heterocyclylthio, heterocyclylcarbonylamino and heterocyclylcarbonyloxy groups, the heterocycles of which may be the groups mentioned above for the 5- or 6-membered heterocyclic group represented by Y. The group bonded through a carbon, nitrogen, oxygen or sulfur atom, represented by Y, may further be selected from substituted amino groups such as mono- and di-substituted amino groups, wherein the substituents may be the lower hydrocarbon, aryl or 5- or 6-membered heterocyclic groups mentioned hereinbefore for Y.

The lower hydrocarbon moiety of the lower hydrocarbon group which may be substituted, as represented by R¹ and R², includes C₁₋₄ alkyl (e.g. methyl, ethyl, propyl, isopropyl), C₂₋₄ alkenyl (e.g. vinyl, 1-methylvinyl, 1-propenyl, allyl, allenyl), C₂₋₄ alkynyl (e.g. ethynyl, 1-propynyl, 2-propynyl), C₃₋₆ cycloalkyl (e.g. cyclopropyl) and so on. The lower hydrocarbon group, R¹ and R², may have 1 or 2 substituents, such as C₁₋₄ alkyl (e.g. methyl, ethyl, propyl, isopropyl), C₂₋₄ alkenyl (e.g. vinyl, 1-methylvinyl, 1-propenyl, allyl, allenyl), C₂₋₄ alkynyl (e.g. ethynyl, 1-propynyl, 2-propynyl), C₃₋₈ cycloalkyl (e.g. cyclopropyl), halogen (e.g. fluorine), hydroxy, oxo, C₁₋₄alkoxy (e.g. methoxy), di-C₁₋₄ alkylamino (e.g. dimethylamino, diethylamino), halo-C₁₋₄ alkyl (e.g. trifluoromethyl), C₁₋₄ acyl (e.g. formyl), hydroxy-C₁₋₄ alkyl (e.g. hydroxymethyl, 2-hydroxyethyl), C₁₋₄ alkoxy-C₁₋₄ alkyl (e.g. methoxymethyl, 2-ethoxyethyl), amino, nitro and so on.

The carboxyl group which may be esterified, as represented by -COOR³ and -COOR⁴, includes carboxy esterified by a C₁₋₅ lower alkyl, benzyl which may be substituted or phenyl which may be substituted, to name but a few examples. The C₁₋₅ lower alkyl mentioned above includes, inter alia, methyl, ethyl, propyl, isopropyl, n-butyl, isobutyl, sec-butyl, tert-butyl, n-pentyl , iso-pentyl, sec-pentyl, neo-pentyl, tert-pentyl and so on. The benzyl which may be substituted includes benzyl and benzyl substituted by 1 to 3 nitro and/or C₁₋₄ alkoxy groups, e.g. nitrobenzyl, methoxybenzyl and so on. The phenyl which may be substituted includes phenyl and phenyl substituted by 1 to 3 nitro and/or C₁₋₄ alkoxy groups, e.g. nitrophenyl, methoxyphenyl and so on.

The following is a partial listing of the preferred species of compound [I].
1) N-[4-(2-Amino-4-hydroxy-7H-pyrrolo[2,3-d]pyrimidin-5-yl)methyl-2-fluorobenzoyl]-L-glutamic acid
2) N-[4-(2-Amino-4-hydroxy-7H-pyrrolo[2,3-d]pyrimidin-5-yl)methyl-2-chlorobenzoyl]-L-glutamic acid
3) N-[4-(2-Amino-4-hydroxy-7H-pyrrolo[2,3-d]pyrimidin-5-yl)methyl-3-fluorobenzoyl]-L-glutamic acid
4) N-[4-(2-Amino-4-hydroxy-7H-pyrrolo[2,3-d]pyrimidin-5-yl)methyl-3-chlorobenzoyl]-L-glutamic acid
5) N-[5-(2-Amino-4-hydroxy-7H-pyrrolo[2,3-d]pyrimidin-5-yl)methyl-2-thenoyl]-L-glutamic acid
6) N-[[5-(2-Amino-4-hydroxy-7H-pyrrolo[2,3-d]pyrimidin-5-yl)methylthiazol-2-yl]carbonyl]-L-glutamic acid
7) N-[4-(4-Hydroxy-2-methyl-7H-pyrrolo[2,3-d]pyrimidin-5-yl)methylbenzoyl]-L-glutamic acid
8) N-[4-(4-Hydroxy-2-methyl-7H-pyrrolo[2,3-d]pyrimidin-5-yl)methyl-2-fluorobenzoyl]-L-glutamic acid
9) N-[4-(4-Hydroxy-2-methyl-7H-pyrrolo[2,3-d]pyrimidin-5-yl)methyl-2-chlorobenzoyl]-L-glutamic acid
10) N-[4-(4-Hydroxy-2-methyl-7H-pyrrolo[2,3-d]pyrimidin-5-yl)methyl-3-fluorobenzoyl]-L-glutamic acid
11) N-[4-(4-Hydroxy-2-methyl-7H-pyrrolo[2,3-d]pyrimidin-5-yl)methyl-3-chlorobenzoyl]-L-glutamic acid
12) N-[5-(4-Hydroxy-2-methyl-7H-pyrrolo[2,3-d]pyrimidin-5-yl)methyl-2-thenoyl]-L-glutamic acid
13) N-[[5-(4-Hydroxy-2-methyl-7H-pyrrolo[2,3-d]pyrimidin-5-yl)methylthiazol-2-yl]carbonyl]-L-glutamic acid
14) N-[4-(2-Amino-4-hydroxy-7H-pyrrolo[2,3-d]pyrimidin-5-yl)methyl-2-fluorobenzoyl]-L-diglutamic acid
15) N-[4-(2-Amino-4-hydroxy-7H-pyrrolo[2,3-d]pyrimidin-5-yl)methyl-2-chlorobenzoyl]-L-diglutamic acid
16) N-[4-(2-Amino-4-hydroxy-7H-pyrrolo[2,3-d]pyrimidin-5-yl)methyl-3-fluorobenzoyl]-L-diglutamic acid
17) N-[4-(2-Amino-4-hydroxy-7H-pyrrolo[2,3-d]pyrimidin-5-yl)methyl-3-chlorobenzoyl]-L-diglutamic acid
18) N-[5-(2-Amino-4-hydroxy-7H-pyrrolo[2,3-d]pyrimidin-5-yl)methyl-2-thenoyl]-L-diglutamic acid
19) N-[[5-(2-Amino-4-hydroxy-7H-pyrrolo[2,3-d]pyrimidin-5-yl)methylthiazol-2-yl]carbonyl]-L-diglutamic acid
20) N-[4-(4-Hydroxy-2-methyl-7H-pyrrolo[2,3-d]pyrimidin-5-yl)methylbenzoyl]-L-diglutamic acid
21) N-[4-(4-Hydroxy-2-methyl-7H-pyrrolo[2,3-d]pyrimidin-5-yl)methyl-2-fluorobenzoyl]-L-diglutamic acid
22) N-[4-(4-Hydroxy-2-methyl-7H-pyrrolo[2,3-d]pyrimidin-5-yl)methyl-2-chlorobenzoyl]-L-diglutamic acid
23) N-[4-(4-Hydroxy-2-methyl-7H-pyrrolo[2,3-d]pyrimidin-5-yl)methyl-3-fluorobenzoyl]-L-diglutamic acid
24) N-[4-(4-Hydroxy-2-methyl-7H-pyrrolo[2,3-d]pyrimidin-5-yl)methyl-3-chlorobenzoyl]-L-diglutamic acid
25) N-[5-(4-Hydroxy-2-methyl-7H-pyrrolo[2,3-d]pyrimidin-5-yl)methyl-2-thenoyl]-L-diglutamic acid
26) N-[[5-(4-Hydroxy-2-methyl-7H-pyrrolo[2,3-d]pyrimidin-5-yl)methylthiazol-2-yl]carbonyl]-L-diglutamic acid
27) N-[4-(2-Amino-4-hydroxy-7H-pyrrolo[2,3-d]pyrimidin-5-yl)methyl-2-fluorobenzoyl]-L-triglutamic acid
28) N-[4-(2-Amino-4-hydroxy-7H-pyrrolo[2,3-d]pyrimidin-5-yl)methyl-2-chlorobenzoyl]-L-triglutamic acid
29) N-[4-(2-Amino-4-hydroxy-7H-pyrrolo[2,3-d]pyrimidin-5-yl)methyl-3-fluorobenzoyl]-L-triglutamic acid
30) N-[4-(2-Amino-4-hydroxy-7H-pyrrolo[2,3-d]pyrimidin-5-yl)methyl-3-chlorobenzoyl]-L-triglutamic acid
31) N-[5-(2-Amino-4-hydroxy-7H-pyrrolo[2,3-d]pyrimidin-5-yl)methyl-2-thenoyl]-L-triglutamic acid
32) N-[[5-(2-Amino-4-hydroxy-7H-pyrrolo[2,3-d]pyrimidin-5-yl)methylthiazol-2-yl]carbonyl]-L-triglutamic acid
33) N-[4-(4-Hydroxy-2-methyl-7H-pyrrolo[2,3-d]pyrimidin-5-yl)methylbenzoyl]-L-triglutamic acid
34) N-[4-(4-Hydroxy-2-methyl-7H-pyrrolo[2,3-d]pyrimidin-5-yl)methyl-2-fluorobenzoyl]-L-triglutamic acid
35) N-[4-(4-Hydroxy-2-methyl-7H-pyrrolo[2,3-d]pyrimidin-5-yl)methyl-2-chlorobenzoyl]-L-triglutamic acid
36) N-[4-(4-Hydroxy-2-methyl-7H-pyrrolo[2,3-d]pyrimidin-5-yl)methyl-3-fluorobenzoyl]-L-triglutamic acid
37) N-[4-(4-Hydroxy-2-methyl-7H-pyrrolo[2,3-d]pyrimidin-5-yl)methyl-3-chlorobenzoyl]-L-triglutamic acid
38) N-[5-(4-Hydroxy-2-methyl-7H-pyrrolo[2,3-d]pyrimidin-5-yl)methyl-2-thenoyl]-L-triglutamic acid
39) N-[[5-(4-Hydroxy-2-methyl-7H-pyrrolo[2,3-d]pyrimidin-5-yl)methylthiazol-2-yl]carbonyl]-L-triglutamic acid
40) Diethyl N-[4-(2-amino-4-hydroxy-7-methylpyrrolo-[2,3-d]pyrimidin-5-yl)methylbenzoyl]-L-glutamate
41) N-[4-(2-Amino-4-hydroxy-7-methylpyrrolo[2,3-d]pyrimidin-5-yl)methylbenzoyl]-L-glutamic acid
42) Diethyl N-[4-(2-amino-4-hydroxy-7H-pyrrolo[2,3-d]pyrimidin-5-yl)methylbenzoyl]-L-glutamate
43) N-[4-(2-amino-4-hydroxy-7H-pyrrolo[2,3-d]pyrimidin-5-yl)methylbenzoyl]-L-glutamic acid
44) Triethyl N-[4-(2-amino-4-hydroxy-7-methylpyrrolo[2,3-d]pyrimidin-5-yl)methylbenzoyl]-L-diglutamate
45) N-[4-(2-Amino-4-hydroxy-7-methylpyrrolo[2,3-d]pyrimidin-5-yl)methylbenzoyl]-L-diglutamic acid
46) Triethyl N-[4-(2-amino-4-hydroxy-7H-pyrrolo[2,3-d]pyrimidin-5-yl)methylbenzoyl]-L-diglutamate
47) N-[4-(2-Amino-4-hydroxy-7H-pyrrolo[2,3-d]pyrimidin-5-yl)methylbenzoyl]-L-diglutamic acid
48) Tetraethyl N-[4-(2-amino-4-hydroxy-7-methylpyrrolo[2,3-d]pyrimidin-5-yl)methylbenzoyl]L-triglutamate
49) N-[4-(2-Amino-4-hydroxy-7-methylpyrrolo[2,3-d]pyrimidin-5-yl)methylbenzoyl]-L-triglutamic acid
50) Tetraethyl N-[4-(2-amino-4-hydroxy-7H-pyrrolo[2,3-d]pyrimidin-5-yl)methylbenzoyl]-L-triglutamate
51) N-[4-(2-Amino-4-hydroxy-7H-pyrrolo[2,3-d]pyrimidin-5-yl)methylbenzoyl]-L-triglutamic acid
52) N-[4-(2-Amino-4-hydroxy-7H-pyrrolo[2,3-d]pyrimidin-5-yl)methylpicolynyl]-L-glutamic acid
53) N-[4-[1-(2-Amino-4-hydroxy-7H-pyrrolo[2,3-d]pyrimidin-5-yl)ethyl]benzoyl]-L-glutamic acid
54) N-[4-[1-(2-Amino-4-hydroxy-7H-pyrrolo[2,3-d]pyrimidin-5-yl)propyl]benzoyl]-L-glutamic acid
55) N-[4-[1-(2-Amino-4-hydroxy-7H-pyrrolo[2,3-d]pyrimidin-5-yl)-2-propenyl]benzoyl]-L-glutamic acid
56) N-[4-[1-(2-Amino-4-hydroxy-7H-pyrrolo[2,3-d]pyrimidin-5-yl)-3-butenyl]benzoyl]-L-glutamic acid
57) N-[4-[1-(2-Amino-4-hydroxy-7H-pyrrolo[2,3-d]pyrimidin-5-yl)-3-butynyl]benzoyl]-L-glutamic acid
58) N-[4-[1-(2-Amino-4-hydroxy-7H-pyrrolo[2,3-d]pyrimidin-5-yl)-2-nitroethyl]benzoyl]-L-glutamic acid
59) N-[4-[1-(2-Amino-4-hydroxy-7H-pyrrolo[2,3-d]pyrimidin-5-yl)-2-aminoethyl]benzoyl]-L-glutamic acid
60) N-[4-[1-(2-Amino-4-hydroxy-7H-pyrrolo[2,3-d]pyrimidin-5-yl)-2-hydroxyethyl]benzoyl]-L-glutamic acid
61) N-[4-[1-(2-Amino-4-hydroxy-7H-pyrrolo[2,3-d]pyrimidin-5-yl)-3-aminopropyl]benzoyl]-L-glutamic acid
62) N-[4-[1-(2-Amino-4-hydroxy-7H-pyrrolo[2,3-d]pyrimidin-5-yl)-3-hydroxypropyl]benzoyl]-L-glutamic acid
63) N-[4-(2-Amino-4-hydroxy-7H-pyrrolo[2,3-d]pyrimidin-5-yl)formylmethylbenzoyl]-L-glutamic acid
64) N-[4-[1-(2-Amino-4-hydroxy-7H-pyrrolo[2,3-d]pyrimidin-5-yl)-2-formylethyl]benzoyl]-L-glutamic acid
65) N-[4-[1-(2-Amino-4-hydroxy-7H-pyrrolo[2,3-d]pyrimidin-5-yl)-3-oxobutanyl]benzoyl]-L-glutamic acid
66) N-[4-[1-(2-Amino-4-hydroxy-7H-pyrrolo[2,3-d]pyrimidin-5-yl)-2-carbamoylethyl]benzoyl]-L-glutamic acid
67) N-[4-[1-(2-Amino-4-hydroxy-7H-pyrrolo[2,3-d]pyrimidin-5-yl)-2-N-methylaminoethyl]benzoyl]-L-glutamic acid
68) N-[4-[1-(2-Amino-4-hydroxy-7H-pyrrolo[2,3-d]pyrimidin-5-yl)-2-N,N-dimethylaminoethyl]benzoyl]-L-glutamic acid
69) N-[4-[1,1-Dimethyl(2-amino-4-hydroxy-7H-pyrrolo [2,3-d]pyrimidin-5-yl)methyl]benzoyl]-L-glutamic acid
70) N-[4-[1,1-Diethyl(2-amino-4-hydroxy-7H-pyrrolo [2,3-d]pyrimidin-5-yl)methyl]benzoyl]-L-glutamic acid
71) N-[5-[1-(2-Amino-4-hydroxy-7H-pyrrolo[2,3-d]pyrimidin-5-yl)ethyl]-2-thenoyl]-L-glutamic acid
72) N-[5-[1-(2-Amino-4-hydroxy-7H-pyrrolo[2,3-d]pyrimidin-5-yl)propyl]-2-thenoyl]-L-glutamic acid
73) N-[5-[1-(2-Amino-4-hydroxy-7H-pyrrolo[2,3-d]pyrimidin-5-yl)-2-propenyl]-2-thenoyl]-L-glutamic acid
74) N-[5-[1-(2-Amino-4-hydroxy-7H-pyrrolo[2,3-d pyrimidin-5-yl)-3-butenyl]-2-thenoyl]-L-glutamic acid
75) N-[5-[1-(2-Amino-4-hydroxy-7H-pyrrolo[2,3-d pyrimidin-5-yl)-3-butynyl]-2-thenoyl]-L-glutamic acid
76) N-[5-[1-(2-Amino-4-hydroxy-7H-pyrrolo[2,3-d]pyrimidin-5-yl)-2-nitroethyl]-2-thenoyl]-L-glutamic acid
77) N-[5-[1-(2-Amino-4-hydroxy-7H-pyrrolo[2,3-d]pyrimidin-5-yl)-2-aminoethyl]-2-thenoyl]-L-glutamic acid
78) N-[5-[1-(2-Amino-4-hydroxy-7H-pyrrolo[2,3-d]pyrimidin-5-yl)-2-hydroxyethyl]-2-thenoyl]-L-glutamic acid
79) N-[5-[1-(2-Amino-4-hydroxy-7H-pyrrolo[2,3-d]pyrimidin-5-yl)-3-aminopropyl]-2-thenoyl]-L-glutamic acid
80) N-[5-[1-(2-Amino-4-hydroxy-7H-pyrrolo[2,3-d]pyrimidin-5-yl)-3-hydroxypropyl]-2-thenoyl]-L-glutamic acid
81) N-[5-(2-Amino-4-hydroxy-7H-pyrrolo[2,3-d]pyrimidin-5-yl)formylmethyl-2-thenoyl]-L-glutamic acid
82) N-[5-[1-(2-Amino-4-hydroxy-7H-pyrrolo[2,3-d]pyrimidin-5-yl)-2-formylmethyl]-2-thenoyl]-L-glutamic acid
83) N-[5-[1-(2-Amino-4-hydroxy-7H-pyrrolo[2,3-d]pyrimidin-5-yl)-3-oxobutanyl]-2-thenoyl]-L-glutamic acid
84) N-[5-[1-(2-Amino-4-hydroxy-7H-pyrrolo[2,3-d]pyrimidin-5-yl)-2-carbamoylethyl]-2-thenoyl]-L-glutamic acid
85) N-[5-[1-(2-Amino-4-hydroxy-7H-pyrrolo[2,3-d]pyrimidin-5-yl)-2-N-methylaminoethyl-]-2-thenoyl]-L-glutamic acid
86) N-[5-[1-(2-Amino-4-hydroxy-7H-pyrrolo[2,3-d]pyrimidin-5-yl)-2-N,N-dimethylaminoethyl]-2-thenoyl]-L-glutamic acid
87) N-[5-[1,1-Dimethyl(2-amino-4-hydroxy-7H-pyrrolo [2,3-d]pyrimidin-5-yl)methyl]-2-thenoyl]-L-glutamic acid
88) N-[5-[1,1-Diethyl(2-amino-4-hydroxy-7H-pyrrolo [2,3-d]pyrimidin-5-yl)methyl]-2-thenoyl]-L-glutamic acid
89) N-[4-(4-Hydroxy-2-methyl-7H-pyrrolo[2,3-d]pyrimidin-5-yl)methylpicolynyl]-L-glutamic acid
90) N-[4-[1-(4-Hydroxy-2-methyl-7H-pyrrolo[2,3-d]pyrimidin-5-yl)ethyl]benzoyl]-L-glutamic acid
91) N-[4-[1-(4-Hydroxy-2-methyl-7H-pyrrolo[2,3-d]pyrimidin-5-yl)propyl]benzoyl]-L-glutamic acid
92) N-[4-[1-(4-Hydroxy-2-methyl-7H-pyrrolo[2,3-d]pyrimidin-5-yl)-2-propenyl]benzoyl]-L-glutamic acid
93) N-[4-[1-(4-Hydroxy-2-methyl-7H-pyrrolo[2,3-d]pyrimidin-5-yl)-3-butenyl]benzoyl]-L-glutamic acid
94) N-[4-[1-(4-Hydroxy-2-methyl-7H-pyrrolo[2,3-d]pyrimidin-5-yl)-3-butynyl]benzoyl]-L-glutamic acid
95) N-[4-[1-(4-Hydroxy-2-methyl-7H-pyrrolo[2,3-d]pyrimidin-5-yl)-2-nitroethyl]benzoyl]-L-glutamic acid
96) N-[4-[1-(4-Hydroxy-2-methyl-7H-pyrrolo[2,3-d]pyrimidin-5-yl)-2-aminoethyl]benzoyl]-L-glutamic acid
97) N-[4-[1-(4-Hydroxy-2-methyl-7H-pyrrolo[2,3-d]pyrimidin-5-yl)-2-hydroxyethyl]benzoyl]-L-glutamic acid
98) N-[4-[1-(4-Hydroxy-2-methyl-7H-pyrrolo[2,3-d]pyrimidin-5-yl)-3-aminopropyl]benzoyl]-L-glutamic acid
99) N-[4-[1-(4-Hydroxy-2-methyl-7H-pyrrolo[2,3-d]pyrimidin-5-yl)-3-hydroxypropyl]benzoyl]-L-glutamic acid
100) N-[4-(4-Hydroxy-2-methyl-7H-pyrrolo[2,3-d]pyrimidin-5-yl)formylmethylbenzoyl]-L-glutamic acid
101) N-[4-[1-(4-Hydroxy-2-methyl-7H-pyrrolo[2,3-d]pyrimidin-5-yl)-2-formylethyl]benzoyl]-L-glutamic acid
102) N-[4-[1-(4-Hydroxy-2-methyl-7H-pyrrolo[2,3-d]pyrimidin-5-yl)-3-oxobutanyl]benzoyl]-L-glutamic acid
103) N-[4-[1-(4-Hydroxy-2-methyl-7H-pyrrolo[2,3-d]pyrimidin-5-yl)-2-carbamoylethyl]benzoyl]-L-glutamic acid
104) N-[4-[1-(4-Hydroxy-2-methyl-7H-pyrrolo[2,3-d]pyrimidin-5-yl)-2-N-methylaminoethyl]benzoyl]-L-glutamic acid
105) N-[4-[1-(4-Hydroxy-2-methyl-7H-pyrrolo[2,3-d]pyrimidin-5-yl)-2-N,N-dimethylaminoethyl]benzoyl]-L-glutamic acid
106) N-[4-[1,1-Dimethyl(4-hydroxy-2-methyl-7H-pyrrolo [2,3-d]pyrimidin-5-yl)methyl]benzoyl]-L-glutamic acid
107) N-[4-[1,1-Diethyl(4-hydroxy-2-methyl-7H-pyrrolo [2,3-d]pyrimidin-5-yl)methyl]benzoyl]-L-glutamic acid
108) N-[5-[1-(4-Hydroxy-2-methyl-7H-pyrrolo[2,3-d]pyrimidin-5-yl)ethyl]-2-thenoyl]-L-glutamic acid
109) N-[5-[1-(4-Hydroxy-2-methyl-7H-pyrrolo[2,3-d]pyrimidin-5-yl)propyl]-2-thenoyl]-L-glutamic acid
110) N-[5-[1-(4-Hydroxy-2-methyl-7H-pyrrolo[2,3-d]pyrimidin-5-yl)-2-propenyl]-2-thenoyl]-L-glutamic acid
111) N-[5-[1-(4-Hydroxy-2-methyl-7H-pyrrolo[2,3-d]pyrimidin-5-yl)-3-butenyl]-2-thenoyl]-L-glutamic acid
112) N-[5-[1-(4-Hydroxy-2-methyl-7H-pyrrolo[2,3-d]pyrimidin-5-yl)-3-butynyl]-2-thenoyl]-L-glutamic acid
113) N-[5-[1-(4-Hydroxy-2-methyl-7H-pyrrolo[2,3-d]pyrimidin-5-yl)-2-nitroethyl]-2-thenoyl]-L-glutamic acid
114) N-[5-[1-(4-Hydroxy-2-methyl-7H-pyrrolo[2,3-d]pyrimidin-5-yl)-2-aminoethyl]-2-thenoyl]-L-glutamic acid
115) N-[5-[1-(4-Hydroxy-2-methyl-7H-pyrrolo[2,3-d]pyrimidin-5-yl)-2-hydroxyethyl]-2-thenoyl]-L-glutamic acid
116) N-[5-[1-(4-Hydroxy-2-methyl-7H-pyrrolo[2,3-d]pyrimidin-5-yl)-3-aminopropyl]-2-thenoyl]-L-glutamic acid
117) N-[5-[1-(4-Hydroxy-2-methyl-7H-pyrrolo[2,3-d]pyrimidin-5-yl)-3-hydroxypropyl]-2-thenoyl]-L-glutamic acid
118) N-[5-(4-Hydroxy-2-methyl-7H-pyrrolo[2,3-d]pyrimidin-5-yl)formylmethyl-2-thenoyl]-L-glutamic acid
119) N-[5-[1-(4-Hydroxy-2-methyl-7H-pyrrolo[2,3-d]pyrimidin-5-yl)-2-formylethyl-2-thenoyl]-L-glutamic acid
120) N-[5-[1-(4-Hydroxy-2-methyl-7H-pyrrolo[2,3-d]pyrimidin-5-yl)-3-oxobutanyl]-2-thenoyl]-L-glutamic acid
121) N-[5-[1-(4-Hydroxy-2-methyl-7H-pyrrolo[2,3-d]pyrimidin-5-yl)-2-carbamoylethyl]-2-thenoyl]-L-glutamic acid
122) N-[5-[1-(4-Hydroxy-2-methyl-7H-pyrrolo[2,3-d]pyrimidin-5-yl)-2-N-methylaminoethyl]-2-thenoyl]-L-glutamic acid
123) N-[5-[1-(4-Hydroxy-2-methyl-7H-pyrrolo[2,3-d]pyrimidin-5-yl)-2-N,N-dimethylaminoethyl]-2-thenoyl]-L-glutamic acid
124) N-[5-[1,1-Dimethyl(4-hydroxy-2-methyl-7H-pyrrolo [2,3-d]pyrimidin-5-yl)methyl]-2-thenoyl]-L-glutamic acid
125) N-[5-[1,1-Diethyl(4-hydroxy-2-methyl-7H-pyrrolo [2,3-d]pyrimidin-5-yl)methyl]-2-thenoyl]-L-glutamic acid

and, diglutamates and triglutamates corresponding to the above glutamic acids of 52) - 125), respectively.

The process for producing the compound [I] or a salt thereof according to the present invention is now described.

The substituent group substituting the N-position of pyrrole or pyrroline in the N-substituted pyrrole or pyrroline ring designated as ring A, includes not only the C₁₋₃ alkyl, C₂₋₃ alkenyl, C₂₋₃ alkynyl, C₃₋₆ cycloalkyl (e.g. cyclopropyl), C₁₋₄ alkanoyl, benzoyl, substituted benzoyl, hydroxy-C₁₋₃ alkyl and C₁₋₃alkoxy-C₁₋₃ alkyl groups mentioned for ring A hereinbefore but also phenyl, substituted phenyl, preferably phenyl substituted by 1 to 3 substituents such as halogen and C₁₋₄ alkoxy (e.g. p-chlorophenyl, p-methoxyphenyl, 3,4,5-trimethoxyphenyl), benzyl, substituted benzyl, preferably benzyl substituted by 1 to 3 halogen, C₁₋₄ alkoxy, and/or phenyl (e.g. p-chlorobenzyl, p-methoxybenzyl , diphenylmethyl) and so on. Among them C₁₋₃ alkyl groups are preferred and methyl is the most desirable.

Ring A' may be bound to 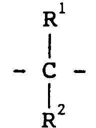
in any available position and may be bound to 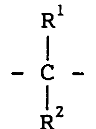
in the N-substitution position (i.e. the N-position) of the pyrrole or pyrroline ring.

The compound [I] or a salt thereof can be produced by acylating an amino acid derivative of general formula [III] with a carboxylic acid of general formula [II] or a reactive derivative of the carboxyl function thereof. The means for this acylation reaction may, for example, comprise acylating a compound [III] with a compound [II] or a reactive derivative thereof. This reaction is preferably conducted in the presence of a carbodiimide, diphenylphosphoryl azide, diethyl cyanophosphonate or the like. The proportion of compound [III] is generally about 1 to 20 moles and preferably about 1 to 5 moles per mole of compound [II] or a reactive derivative thereof. The amount of said carbodiimide, diphenylphosphoryl azide, diethyl cyanophosphonate or the like is generally about 1 to 25 moles and preferably about 1 to 5 moles per mole of compound [II] or a salt thereof. The carbodiimide is preferably dicyclohexylcarbodiimide for practical purposes, although such other carbodiimides can also be employed as, for example, diphenylcarbodiimide, di-o-tolylcarbodiimide, di-p-tolylcarbodiimide, di-tert-butylcarbodiimide, 1-cyclohexyl-3-(2-morpholinoethyl)carbodiimide, 1-cyclohexyl-3-(4-diethylaminocyclohexyl)carbodiimide, 1-ethyl-3-(2-diethylaminopropyl)carbodiimide and 1-ethyl-3-(3-diethylaminopropyl)carbodiimide. This acylation reaction is preferably carried out in the presence of an appropriate solvent. The solvent for this purpose includes, inter alia, water, alcohols (e.g. methanol, ethanol), ethers (e.g. dimethyl ether, diethyl ether, tetrahydrofuran, dioxane, monoglyme, diglyme), nitriles (e.g. acetonitrile), esters (e.g. ethyl acetate), halogenated hydrocarbons (e.g. dichloromethane, chloroform, carbon tetrachloride), aromatic hydrocarbons (e.g. benzene, toluene, xylene), acetone, nitromethane, pyridine, dimethyl sulfoxide, N,N-dimethylformamide, hexamethylphosphoramide, sulfolane, etc., or appropriate mixtures thereof. This reaction is conduced generally at pH about 2 to 14 and preferably in the range of pH about 6 to 9. Usually, the reaction is carried out at a temperature between about -10°C and the boiling point of the reaction solvent (about 100°C) and preferably in the range of about 0°C to 50°C. The reaction time may range from about 1 to 100 hours. The pH of the reaction mixture is adjusted, where necessary, with an acid (e.g. hydrochloric acid, sulfuric acid, phosphoric acid, nitric acid, acetic acid), a base (e.g. sodium methoxide, sodium ethoxide, sodium hydroxide, potassium hydroxide, barium hydroxide, lithium hydroxide, sodium carbonate, potassium carbonate, barium carbonate, calcium carbonate, sodium hydrogen carbonate, trimethylamine, triethylamine, triethanolamine, pyridine) or a buffer (e.g. phosphate buffer, borate buffer, acetate buffer). This reaction can be accelerated by using a catalyst capable of promoting acylation.

The catalyst mentioned above may for example be a base catalyst or an acid catalyst. The base catalyst includes, inter alia, tertiary amines (e.g. aliphatic tertiary amines such as triethylamine and aromatic tertiary amines such as pyridine, α-, β- or γ-picoline, 2,6-lutidine, 4-dimethylaminopyridine, 4-(1-pyrrolidinyl)pyridine, dimethylaniline and diethylaniline). The acid catalyst includes, inter alia, Lewis acids [e.g. anhydrous zinc chloride, anhydrous aluminum chloride (AlCl₃), anhydrous ferric chloride, titanium tetrachloride (TiCl₄), tin tetrachloride (SnCl₄), antimony pentachloride, cobalt chloride, cupric chloride, boron trifluoride etherate, etc.]. Among the above catalysts, 4-dimethylaminopyridine or 4-(1-pyrrolidinyl)pyridine is preferred in many cases. The amount of the catalyst may be a catalytic amount necessary for acceleration of acylation reaction and is generally about 0.01 to 10 moles and preferably about 0.1 to 1 mole per mole of compound [II] or a salt thereof. The reactive derivative of the carboxyl function of carboxylic acid [II] includes, inter alia, acid halides (e.g. fluoride, chloride, bromide, iodide), acid anhydrides (e.g. iodoacetic anhydride, isobutyric anhydride), mixed acid anhydride with lower monoalkyl carbonates (e.g. monomethyl carbonate, monoethyl carbonate, monopropyl carbonate, monoisopropyl carbonate, monobutyl carbonate, mono-iso-butyl carbonate, mono-sec-butyl carbonate, mono-tert-butyl carbonate), mixed acid anhydries with active esters (e.g. cyanomethyl ester, ethoxycarbonylmethyl ester, methoxymethyl ester, phenyl ester, o-nitrophenyl ester, p-nitrophenyl ester, p-carbomethoxyphenyl ester, p-cyanophenyl ester, phenylthio ester, succinimide ester), acid azides, phosphoric diesters (e.g. dimethyl phosphate, diethyl phosphate, dibenzyl phosphate, diphenyl phosphate), and mixed acid anhydries with phosphorous diesters (e.g. dimethyl phosphite, diethyl phosphite, dibenzyl phosphite, diphenyl phosphite). The solvent, catalyst, reaction temperature and time and other conditions for the acylation reaction using such a reactive derivative are the same as those of acylation in the presence of said carbodiimide.

For the production of compounds or salts thereof, among said compounds [I] and salts thereof, which contain hydroxy, amino, mercapto or carboxy as the substituent of B, Y, R¹ or R², or the production of compounds or salts in which -COOR³ and -COOR⁴ are carboxy, an alternative procedure comprises protecting said hydroxy, amino, mercapto or carboxy group with a per se known protective group, then reacting a compound of general formula [II] or a reactive derivative of the carboxyl function thereof with a compound of general formula [III], and finally removing the protective group in the per se known manner to give the object compound [I] or a salt thereof.

The starting compound [II] or a salt or ester thereof is a novel compound which can be produced by the known processes described in the literature [e.g. J. A. Secrist III and P. S. Liu, Journal of Organic Chemistry, 43, 3937-3941 (1978); B. Roth, R. Laube, M. Y. Tidwell, and B. S. Rauckman, Journal of Organic Chemistry, 45, 3651-3657 (1980); USP4,997,838, EP-A-438,261; USP4,996,206; USP5,028,608, etc.

The compound [II] can be used in the form of a salt. The salt with a base includes the salts with alkali metals, alkaline earth metals, nontoxic metals, ammonium, substituted ammonium and so on. To be specific, there may be mentioned the salts with sodium, potassium, lithium, calcium, magnesium, aluminum, zinc, ammonium, trimethylammonium, triethylammonium, triethanolammonium, pyridinium, substituted pyridinium and so on. The salt with an acid includes the salts with mineral acids such as hydrochloric acid, sulfuric acid, nitric acid, phosphoric acid, boric acid, etc., and salts with organic acids such as oxalic acid, tartaric acid, acetic acid, trifluoroacetic acid, methanesulfonic acid, benzenesulfonic acid, p-toluenesulfonic acid, camphorsulfonic acid and so on.

The ester of compound [II] includes, inter alia, the esters mentioned for -COOR³ and -COOR⁴ hereinbefore.

The starting compound [III] can be produced by per se known processes or processes analogous therewith. For example, the processes described in-the literature [e.g. J. P. Greenstein and M. Winitz, Chemistry of the Amino Acids Vol. 1-3, John Wiley & Sons, Inc., New York London (1961)] may be followed.

The compound [I] produced in the above manner can be isolated from the reaction mixture by the conventional separatory procedures such as concentration, solvent extraction, chromatography and recrystallization.

The compound [I] obtainable by the production process described hereinbefore may be in the salt form. The salt with a base includes, inter alia, salts with alkali metals, alkaline earth metals, nontoxic metals, ammonium, substituted ammonium and so on. To be specific, there may be mentioned the salts with sodium, potassium, lithium, calcium, magnesium, aluminum, zinc, ammonium, trimethylammonium, triethylammonium, triethanolammonium, pyridinium, substituted pyridinium and so on. The salt with an acid'includes salts with mineral acids such as hydrochloric acid, sulfuric acid, nitric acid, phosphoric acid, boric acid, etc., and salts with organic acids such as oxalic acid, tartaric acid, acetic acid, trifluoroacetic acid, methanesulfonic acid, benzenesulfonic acid, p-toluenesulfonic acid, camphorsulfonic acid and so on.

The compound [I] or its salt has excellent inhibitory activity against thymidylate synthase which utilizes thymidylate and its congeners as substrates. Therefore, these compounds can be used, either singly or in combination with other antitumor agents, in the treatment of villous carcinoma, leukemia, mammary adenocarcinoma, epidermoid carcinoma of head and neck, squamous cell carcinoma lymphosarcoma and small cell lung carcinoma in which MTX has heretofore been indicated and even for the purpose of treating various MTX-resistant tumors. For example, the compound [I] and its salt not only exhibit high antitumoral activity against mouse tumor cell lines (P388, L1210, L5178Y, B16 melanoma, Meth A, Lewis Lung Carcinoma, S180 sarcoma, Erhlich Carcinoma, Colon26 and 38, etc.) and human tumor cell lines (A549, HL60, KB, etc.) but also have activity to reduce tumors [e.g. leukemia, melanoma, sarcoma, mastocytoma, carcinoma, neoplasia, etc.] in warm-blooded animals and can be used as safe antitumor agents in the treatment of tumors in tumor-bearing warm-blooded animals and particularly in mammals (e.g. human, mouse, rat, cat, dog, rabbit).

For use as an antitumor drug, compound [I] or a pharmaceutically acceptable salt thereof, either alone or as formulated with a pharmaceutically acceptable carrier, vehicle or diluent in the conventional manner, can be administered orally or otherwise in various forms such as powder, granule, tablet, capsule, suppository, injection and so on. While the dosage depends on the recipient animal species, disease and condition to be treated, species of compound, route of administration, etc. , the dosage for oral administration is about 1.0 to 500 mg and preferably about 10.0 to 200 mg, as the compound of the invention, per day per kg body weight of the warm-blooded animal and that for non-oral administration is about 1.0 to 200 mg and preferably 5.0 to 100 mg, as the compound of the invention, per day per kg body weight.

The pharmaceutical formulation referred to above can be accomplished in accordance with the per se known procedures. For the manufacture of an oral preparation, for example a tablet, there can be incorporated a binder (e.g. hydroxypropylcellulose, hydroxypropylmethylcellulose, macrogols, etc.), a disintegrator (e.g. starch, carboxymethylcellulose calcium, etc.), a lubricant (e.g. magnesium stearate, talc, etc.) in suitable proportions. For the manufacture of a non-oral preparation, for example an injection, there can be employed an isotonizing agent (e.g. glucose, D-sorbitol, D-mannitol, sodium chloride, etc.), a preservative (e.g. benzyl alcohol, chlorobutanol, methyl p-hydroxybenzoate, propyl p-hydroxybenzoate, etc.), a buffer (e.g. phosphate buffer, sodium acetate buffer, etc.) in suitable proportions.

The manufacture of said tablet is now described by way of example. About 1.0 to 50 mg of compound [I] or salt, 100-500 mg of lactose, about 50-100 mg of corn starch and about 5 to 20 mg of hydroxypropylcellulose, all per tablet, are blended and granulated. After addition of corn starch and magnesium stearate, the granules are compression-molded into a tablet weighing about 100-500 mg and having a diameter of about 3-100 mm. When this tablet is coated with a coating composition prepared by dissolving hydroxypropylmethylmethylcellulose phthalate (about 10-20 mg) and castor oil (about 0.5-2.0 mg) at a final concentration of about 5-10% in acetone-ethanol, an enteric-coated tablet is obtained. The manufacture of an injection is now described by way of example. About 2.0-50 mg, per ampule, of the sodium salt of compound [I] is dissolved in about 2 ml of physiological saline and (1) the solution is filled into an ampule and, after sealing, sterilized by heating at about 110°C for about 30 minutes or (2) is dissolved in a vehicle prepared by dissolving about 10-40 mg of mannitol or sorbitol in about 2 ml of distilled water, lyophilized and sealed. The lyophilized product is unsealed, dissolved by adding physiological saline or the like to give a ca. 1.0-50 mg/ml solution which can be administered subcutaneously, intravenously or intramuscularly.

The pharmacological activities of Compound (I) or its salts of the invention are shown depending upon experiments below.

The thymidylate synthase (TS) inhibitory activity and in vitro antiproliferative activity against Meth A fibrosarcoma cells of the representative subject compounds obtained in Examples described hereinafter were determined by the following methods.

### Experiment 1

### Assay of TS inhibitory activity

A crude fraction of TS was prepared from Meth A fibrosarcoma (Meth A) cells subcultured in vitro. For cell culture, Eagle's minimum essential medium (MEM, Nissui Pharmaceutical) containing 10% fetal bovine serum was used. Cells in the logarithmic growth phase were harvested, washed twice with phosphate buffered saline, suspended in 0.2 M sucrose and 0.01 M Tris-HCl buffer (pH 7.5), ultrasonicated, and centrifuged at 100,000 x g to obtain a supernatant. The protein concentration was determined using a protein dye reagent (Bio-Rad) with bovine γ-globulin as standard protein. Samples with a protein concentration of 100 mg/ml were prepared.

The enzymatic reaction was conducted as follows, using the method described by D. Roberts in Biochemistry 5, 3546 (1966), partially modified. The reaction mixture consisted of 0.058% formaldehyde, 6.78 mM sodium fluoride, 0.2 mM 2-mercaptoethanol, 6.24 mg/ml bovine serum albumin, 80 µM 2'-deoxy-5'-monophosphate (dUMP), 80 µM tetrahydrofolic acid, 2 mg/ml crude TS fraction, and 0.173 M Tris-HCl buffer (pH 7.5). To this mixture was added a varying concentration of each Example compound. To 50 µl of each final mixture was added 540 KBq [H³]dUMP and the resultant mixture was incubated in a 96-well plate at 37°C for 1 hour. After completion of the incubation, 26.65% trichloroacetic acid and 3.33 mg/ml dUMP were added to stop the reaction and 220 µl of 11.4 mg/ml activated charcoal was added. The whole volume was centrifuged and the radioactivity in 100 µl of the supernatant was determined by the liquid scintillation method. Thus, the concentration required to cause 50% inhibition of TS activity (IC₅₀ value) was obtained. The results are shown in Table 1.

### Experiment 2

### Assay of antiproliferative activity against Meth A fibrosarcoma (Meth A) cells

Meth A cells (2 x 10⁴ cells/ml) prepared in the routine manner were seeded, 2.0 ml per well, in a 12-well plate and subjected to stationary culture at 37°C and 5% CO₂. Each Example compound dissolved at an appropriate concentration was serially diluted 1:2 to 1:10 with MEM (Nissui Pharmaceutical) and added in the medium and again the cells were subjected to stationary culture for 72 hours at 37°C and 5% CO₂. Then, the total cell count at each concentration was determined with a Coulter counter (Coulter Electronics, FL) and the mean value of 3 wells was expressed as cell count per milliliter. The concentration of each compound required to reduce the cell count of the untreated control group to 50% was regarded as the IC₅₀ value of the compound. The results are shown in Table 2.

**Table 1**

| TS inhibitory activity | |
|---|---|
| Compound | IC₅₀ (µM) |
| Example 2 | 0.86 |
| Example 6 | 0.083 |
| Example 8 | 0.49 |
| Example 10 | 0.039 |
| Example 12 | |
| Example 14 | 0.037 |
| Example 16 | 0.59 |

**Table 2**

| Meth A cell growth inhibitory activity | |
|---|---|
| Compound | IC₅₀ (µM) |
| Example 4 | 0.076 |
| Example 6 | 0.063 |
| Example 8 | 0.11 |
| Example 10 | 0.066 |
| Example 12 | 0.039 |
| Example 14 | 0.091 |
| Example 16 | 0.093 |
| Example 18 | 0.81 |

From the above Table 1 and Table 2, it is clear that the Compound (I) or its salts have excellent activities in TS inhibition and Meth A cell growth inhibition.

The following examples are intended to describe the invention in further detail.

### Example 1

Production of diethyl N-[4-(2-amino-4-hydroxy-7-methylpyrrolo[2,3-d]pyrimidin-5-yl)methylbenzoyl]-L-glutamate

To a solution of t-butyl 4-(2-amino-4-hydroxy-7-methylpyrrolo[2,3-d]pyrimidin-5-yl)methylbenzoate (2.35 g) in dichloromethane (50 ml) was added trifluoroacetic acid (10 ml) and the mixture was stirred at room temperature for 4 hours. The solvent was distilled off under reduced pressure and the residue and diethyl glutamate hydrochloride (1.90 g) were dissolved in dry N,N-dimethylformamide (DMF; 200 ml). After the reaction mixture was cooled to 0°C, a solution of diethyl cyanophosphonate (1.30 g) in dry DMF (25 ml) was added. The mixture was stirred for 15 minutes. Then, a solution of triethylamine (5.72 g) in dry DMF (25 ml) was added and the mixture was stirred at 0°C for 1 hour and further at room temperature for 18 hours. The solvent was then distilled off under reduced pressure and the residue was purified by silica gel column chromatography [carrier: 250 g, eluent: chloroform-10% ammonia/ethanol = 9.5:0.5] to give the title compound (2.15 g).
¹H-NMR (Me₂SO-d₆) δ: 1.17 (3H, t, J=7 Hz), 1.19 (3H, t, J=7 Hz), 1.92-2.17 (2H, m), 2.43 (2H, t, J=8 Hz), 3.43 (3H, s), 3.99 (2H, s), 4.05 (2H, q, J=7 Hz), 4.11 (2H, q, J=7 Hz), 4.37-4.49 (1H, m), 6.17 (2H, s), 6.34 (1H, s), 7.38 (2H, d, J=8 Hz), 7.77 (2H, d, J=8 Hz), 8.60 (1H, d, J=8 Hz), 10.18 (1H, s)
IR (KBr): 3340, 3150, 2990, 2940, 1740, 1690 cm⁻¹

### Example 2

Production of N-[4-(2-amino-4-hydroxy-7-methylpyrrolo-[2,3-d]pyrimidin-5-yl)methylbenzoyl]-L-glutamic acid

The compound of Example 1 (2.12 g) was dissolved in water (90 ml)-tetrahydrofuran (60 ml) followed by addition of 1N aqueous sodium hydroxide solution (13.1 ml), and the mixture was stirred at room temperature for 4 hours. The tetrahydrofuran was distilled off under reduced pressure and the residue was filtered through a Milipore filter to remove a small amount of impurity. To the filtrate was added IN hydrochloric acid (13.1 ml), whereupon a white powder separated out. The power was collected by filtration and dried to give the title compound (1.51 g).
¹H-NMR (Me₂SO-d₆) δ: 1.80-2.20 (2H, m), 2.34 (2H, t, J=7 Hz), 3.42 (3H, s), 3.98 (2H, s), 4.29-4.46 (1H, m), 6.16 (2H, s), 6.32 (2H, s), 7.37 (2H, d, J=8 Hz), 7.76 (2H, d, J=8 Hz), 8.47 (1H, d, J=8 Hz), 10.18 (1H, s)
IR (KBr): 3400, 3300, 3150, 2930, 1700, 1680, 1525, 1500 cm⁻¹

### Example 3

Production of diethyl N-[4-(2-amino-4-hydroxy-7H-pyrrolo[2,3-d]pyrimidin-5-yl)methylbenzoyl]-L-glutamate

The procedure of Example 1 was followed starting with t-butyl 4-[2-amino-4-hydroxy-7H-pyrrolo[2,3-d]pyrimidin-5-yl]methylbenzoate (1.39 g) to give the title compound (1.70 g).

¹H-NMR (Me₂SO-d₆) δ: 1.16 (3H, t, J=7 Hz), 1.18 (3H, t, J=7 Hz), 1.91-2.20 (2H, m), 2.42 (2H, t, J=7 Hz), 3.99 (2H, s), 4.04 (2H, q, J=7 Hz), 4.10 (2H, q, J=7 Hz), 4.37-4.47 (1H, m), 5.99 (2H, s), 6.32 (1H, s), 7.37 (2H, d, J=8 Hz), 7.74 (2H, d, J=8 Hz), 8.58 (1H, d, J=7 Hz), 10.11 (1H, s), 10.71 (1H, s)
IR (KBr): 3430, 3280, 3150, 2980, 1735, 1710, 1675, 1650, 1620, 1580, 1540, 1430, 1375, 1300, 1250, 1190, 1090, 1020 cm⁻¹

### Example 4

Production of N-[4-(2-amino-4-hydroxy-7H-pyrrolo[2,3-d]pyrimidin-5-yl)methylbenzoyl]-L-glutamic acid

The procedure of Example 2 was followed starting with the compound of Example 3 (1.60 g) to give the title compound (1.39 g).
¹H-NMR (Me₂SO-d₆) δ: 1.85-2.20 (2H, m), 2.34 (2H, t, J=7 Hz), 3.98 (2H, s), 4.34-4.47 (1H, m), 5.99 (2H, s), 6.32 (1H, s), 7.37 (2H, d, J=8 Hz), 7.75 (2H, d, J=8 Hz), 8.46 (1H, d, J=8 Hz), 10.13 (1H, s), 10.71 (1H, s)
IR (KBr): 3320, 2930, 1700, 1650, 1535, 1500, 1430, 1400, 1340, 1220, 1180, 1070 cm⁻¹

### Example 5

Production of diethyl N-[5-(2-amino-4-hydroxy-7H-pyrrolo [2,3-d)pyrimidin-5-yl)methyl-2-thenoyl]-L-glutamate

The procedure of Example 1 was followed starting with t-butyl 5-(2-amino-4-hydroxy-7H-pyrrolo[2,3-d)pyrimidin-5-yl)methyl-2-thiophenecarboxylate (800 mg) to give the title compound (1.095 g).
¹H-NMR(Me₂SO-d₆) δ:1.16 (3H,t,J=7 Hz), 1.20 (3H,t,J=7 Hz), 1.89-2.22 (2H,m), 2.40 (2H,t,J=7 Hz), 4.04 (2H,q,J=7 Hz), 4.10 (2H,q,J=7 Hz), 4.13 (2H,s), 4.24-4.41 (1H,m), 6.10 (2H,s), 6.45 (1H,s), 6.91 (1H,d,J=4 Hz), 7.65 (1H,d,J=4 Hz), 8.58 (1H,d,J=8 Hz), 10.22 (1H,s), 10.82 (1H,s)
IR(KBr): 3320, 2980, 1735, 1700cm⁻¹

### Example 6

Production of N-[5-(2-amino-4-hydroxy-7H-pyrrolo[2,3-d]pyrimidin-5-yl)methyl-2-thenoyl]-L-glutamic acid

The procedure of Example 2 was followed starting with the compound of Example 5 (1.09 g) to give the title compound (416 mg).
¹H-NMR (Me₂SO-d₆) δ: 1.81-2.19 (2H,m), 2.22-2.47 (2H,m), 4.14 (2H,s), 4.24-4.41 (1H,m), 6.05 (2H,s), 6.45 (1H,s), 6.91 (1H,d,J=3 Hz), 7.65 (1H,d,J=3 Hz), 8.45 (1H,d,J=7 Hz), 10.1,8 (1H,s), 10.81 (1H,s)
IR(KBr): 3350, 3240, 2930, 1700-1640, 1540cm⁻¹

### Example 7

Production of diethyl N-[4-(2-amino-4-hydroxy-7H-pyrrolo[2,3-d]pyrimidin-5-yl)methyl-3-fluorobenzoyl]-L-glutamate

Methyl 4-(2-amino-4-hydroxy-7H-pyrrolo[2,3-d]pyrimidin-5-yl)methyl-3-fluorobenzoate (803 mg) was dissolved in water (10 ml)-tetrahydrofuran (15 ml)-methanol (2 ml), followed by addition of 1N aqueous sodium hydroxide solution (8.89 ml), and the mixture was stirred at room temperature for 2.5 hours. The tetrahydrofuran and methanol were distilled off under reduced pressure, followed by addition of 8.89 ml of 1N hydrochloric acid to precipitate a white powder. This white powder was collected by filtration and dried, and the thus obtained white powder and diethyl glutamate hydrochloride (630 mg) were dissolved in dry DMF (50 ml). After the reaction mixture was cooled to 0°C, a solution of diethyl cyanophosphonate (428 mg) in dry DMF (5 ml) was added. The mixture was stirred for 15 minutes. Then, a solution of triethylamine (886 mg) in dry DMF (5 ml) was added, and the mixture was stirred at 0°C for 1 hour and further at room temperature for 18 hours. The solvent was then distilled off under reduced pressure and the residue was purified by silica gel column chromatography [carrier: 70 g, eluent: chloroform-10 % ammonia/ethanol = 9.5 : 0.5] to give the title compound (1.02 g).
¹H-NMR (Me₂SO-d₆) δ: 1.17 (3H,t,J=7 Hz), 1.19 (3H,t,J=7 Hz), 1.92-2.21 (2H,m), 2.46 (2H,t,J=5 Hz), 4.01 (2H,q,J=7 Hz), 4.05 (2H,q,J=7 Hz), 4.07 (2H,s), 4.34-4.49 (1H,m), 6.03 (2H,s), 6.28 (1H,s). 7.40 (1H,t,J=8 Hz), 7.57-7.66 (2H,m), 8.72 (1H,d,J=8 Hz), 10.16 (1H,s), 10.78 (1H,s)
IR (KBr): 3320, 2980, 1740, 1670, 1640cm⁻¹

### Example 8

Production of N-[4-(2-amino-4-hydroxy-7H-pyrrolo[2,3-d]pyrimidin-5-yl)methyl-3-fluorobenzoyl]-L-glutamic acid

The procedure of Example 2 was followed starting with the compound of Example 7 (1.02 g) to give the title compound (759 mg).
¹H-NMR (Me₂SO-d₆) δ: 1.82-2.19 (2H,m), 2.35 (2H,t,J= 7 Hz), 4.02 (2H,s), 4.33-4.47 (1H,m), 6.05 (2H,s), 6.27 (1H,s), 7.40 (1H,t,J=8 Hz), 7.59-7.67 (2H,m), 8.59 (1H,d,J=7 Hz), 10.18 (1H,s), 10.78 (1H,s)
IR (KBr): 3350, 2930, 1710-1540cm⁻¹

### Example 9

Production of diethyl N-[4-[1-(2-amino-4-hydroxy-7H-pyrrolo[2,3-d]pyrimidin-5-yl)ethyl]benzoyl]-L-glutamate

The procedure of Example 1 was followed starting with t-butyl 4-[1-(2-amino-4-hydroxy-7H-pyrrolo[2,3-d]pyrimidin-5-yl)ethyl]benzoate (800 mg) to give the title compound (974 mg).
¹H-NMR (Me₂SO-d₆) δ: 1.16 (3H,t,J=7.2 Hz), 1.18 (3H,t,J=7.2 Hz), 1.55 (3H,d,J=7.4 Hz), 1.94-2.18 (2H,m), 2.42 (2H,t,J=7.4 Hz), 4.05 (2H,q,J=7.2 Hz), 4.10 (2H,q,J=7.2 Hz), 4.30-4.49 (2H,m), 5.98 (2H,s), 6.34 (1H,d,J=1.6 Hz), 7.39 (2H,d,J=8.4 Hz), 7.75 (2H,d,J=8.4 Hz), 8.58 (1H,d,J=7.6 Hz), 10.05 (1H,s), 10.71 (1H,d J=1.6 Hz)
IR (KBr): 3350, 2970, 2920, 1735, 1680-1580, 1530, 1500cm⁻¹

### Example 10

Production of N-[4-[1-(2-amino-4-hydroxy-7H-pyrrolo[2,3-d]pyrimidin-5-yl)ethyl]benzoyl]-L-glutamic acid

The procedure of Example 2 was followed starting with the compound of Example 9 (920 mg) to give the title compound (674 mg).
¹H-NMR (Me₂SO-d₆) δ: 1.55 (3H,d,J=7.2 Hz), 1.88-2.18 (2H,m), 2.34 (2H,t,J=7.4 Hz), 4.31-4.47 (2H,m), 5.98 (2H,s), 6.32 (1H,s), 7.38 (2H,d,J=8.2 Hz), 7.75 (2H,d,J=8.2 Hz), 8.45 (1H,d,J=8.0 Hz), 10.04 (1H,s), 10.70 (1H,s)
IR (KBr): 3350, 3200, 2960, 1700, 1660cm⁻¹

### Example 11

Production of diethyl N-[4-[1-(2-amino-4-hydroxy-7H-pyrrolo[2,3-d]pyrimidin-5-yl)propyl]benzoyl]-L-glutamate

The procedure of Example 1 was followed starting with t-butyl 4-[1-(2-amino-4-hydroxy-7H-pyrrolo[2,3-d]pyrimidin-5-yl)propyl]benzoate (500 mg) to give the title compound (528 mg).
¹H-NMR (Me₂SO-d₆) δ: 0.81 (3H,t,J=7.0 Hz), 1.16 (3H,t,J=7.0 Hz), 1.18 (3H,t,J=7.0 Hz), 1.84-2.23 (4H,m), 2.42 (2H,t,J=7.4 Hz), 4.04 (2H,q,J=7.0 Hz), 4.10 (2H,q,J=7.0 Hz), 4.11-4.19 (1H,m), 4.35-4.49 (1H,m), 5.98 (2H,s), 6.40 (1H,d,J=1.4 Hz), 7.41 (2H,d,J=8.4 Hz), 7.74 (2H,d,J=8.4 Hz), 8.58 (1H,d,J=7.8 Hz), 10.06 (1H,s), 10.72 (1H,d,J=1.4 Hz)
IR(KBr): 3425, 3300, 3240, 3150, 2960, 2925, 2870, 1730, 1680, 1650, 1625, 1600cm⁻¹

### Example 12

Production of N-[4-[1-(2-amino-4-hydroxy-7H-pyrrolo[2,3-d]pyrimidin-5-yl)propyl]benzoyl]-L-glutamic acid

The procedure of Example 2 was followed starting with the compound of Example 11 (513 mg) to give the title compound (292 mg).
¹H-NMR (Me₂SO-d₆) δ: 0.80 (3H,t,J=7.4 Hz), 1.81-2.21 (4H,m), 2.34 (2H,t,J=7.4 Hz), 4.14 (1H,t,J=7.4 Hz), 4.31-4.45 (1H,m), 5.97 (2H,s), 6.39 (1H,s), 7.40 (2H,d,J=8.2 Hz), 7.74 (2H,d,J=8.2 Hz), 8.45 (1H,d,J=7.8 Hz), 10.05 (1H,s), 10.71 (1H,s)
IR(KBr): 3350, 2960, 2920, 2870, 1700-1600, 1530cm⁻¹

### Example 13

Production of diethyl N-[4-[1-(2-amino-4-hydroxy-7H-pyrrolo[2,3-d]pyrimidin-5-yl)-2-propenyl]benzoyl]-L-glutamate

The procedure of Example 1 was followed starting with t-butyl 4-[1-(2-amino-4-hydroxy-7H-pyrrolo[2,3-d]pyrimidin-5-yl)-2-propenyl]benzoate (165 mg) to give the title compound (171 mg).
¹H-NMR (Me₂SO-d₆) δ: 1.16 (3H,t,J=7.0 Hz), 1.18 (3H,t,J=7.0 Hz), 1.92-2.16 (2H,m), 2.42 (2H,t,J=7.4 Hz), 4.04 (2H,q,J=7.0 Hz), 4.09 (2H,q,J=7.0 Hz), 4.33-4.48 (1H,m), 4.99 (1H,d,J=16.4 Hz), 5.04 (1H,d,J=10.0 Hz), 5.01-5.07 (1H,m), 6.00 (2H,s), 6.43 (1H,d,J=1.6 Hz), 6.44 (1H,ddd,J=8.2 Hz, 10.0 Hz,16.4 Hz), 7.31 (2H,d,J=8.4 Hz), 7.74 (2H,d,J=8.4 Hz), 8.58 (1H,d,J=7.6 Hz), 10.07 (1H,s), 10.81 (1H,d,J=1.6 Hz)
IR(KBr): 3350, 3215, 2980, 1740, 1680-1600, 1540, 1500cm⁻¹

### Example 14

Production of N-[4-[1-(2-amino-4-hydroxy-7H-pyrrolo[2,3-d]pyrimidin-5-yl)-2-propenyl]benzoyl]-L-glutamic acid

The procedure of Example 2 was followed starting with the compound of Example 13 (146 mg) to give the title compound (82 mg).
¹H-NMR (Me₂SO-d₆) δ: 1.87-2.18 (2H,m), 2.34 (2H,t,J=7.2 Hz), 4.32-4.45 (1H,m), 4.93-5.12 (3H,m), 6.00 (2H,s), 6.34-6.53 (1H,m), 6.44 (1H,s), 7.31 (2H,d,J=8.0 Hz), 7.75 (2H,d,J=8.0 Hz), 8.46 (1H,d,J=7.8 Hz), 10.07 (1H,s), 10.81 (1H,s)
IR(KBr): 3350, 2940, 1700-1600, 1540cm⁻¹

### Example 15

Production of diethyl N-[4-[1-(2-amino-4-hydroxy-7H-pyrrolo[2,3-d]pyrimidin-5-yl)-2-nitroethyl]benzoyl]-L-glutamate

The procedure of Example 1 was followed starting with t-butyl 4-[1-(2-amino-4-hydroxy-7H-pyrrolo[2,3-d]pyrimidin-5-yl)-2-nitroethyl]benzoate (131 mg) to give the title compound (151 mg).
¹H-NMR (Me₂SO-d₆) δ: 1.18 (3H,t,J=7 Hz), 1.19 (3H,t,J=7 Hz), 1.86-2.13 (2H,m), 2.42 (2H,t,J=7 Hz), 4.04 (2H,q,J=7 Hz), 4.10 (2H,q,J=7 Hz), 4.36-4.48 (1H,m), 5.04 (1H,d,J=8 Hz), 5.30-5.57 (2H,m), 6.14 (2H,s), 6.52 (1H,d,J=2 Hz), 7.55 (2H,d,J=8 Hz) 7.77 (2H,d,J=8 Hz), 8.65 (1H,d,J=7 Hz), 10.34 (1H,s), 10.93 (1H,d,J=2 Hz)
IR(KBr): 3300, 2980, 1735, 1680-1640, 1550cm⁻¹

### Example 16

Production of N-[4-[1-(2-amino-4-hydroxy-7H-pyrrolo[2,3-d]pyrimidin-5-yl)-2-nitroethyl]benzoyl]-L-glutamic acid

The procedure of Example 2 was followed starting with the compound of Example 15 (141 mg) to give the title compound (43 mg).
¹H-NMR (Me₂SO-d₆) δ: 1.83-2.19 (2H,m), 2.34-2.47 (2H,m), 4.29-4.41 (1H, m), 5.03 (1H,t,J=8 Hz), 5.28-5.54 (2H,m), 6.10 (2H,s), 6.52 (1H,s), 7.54 (2H,d,J=8 Hz), 7.77 (2H,d,J=8 Hz), 8.51 (1H,d,J=8 Hz), 10.29 (1H,s), 10.92 (1H,s)
IR(KBr): 3350, 3230, 2940, 1700-1600, 1550cm⁻¹

### Example 17

Production of diethyl N-[4-[1-(2-amino-4-hydroxy-7H-pyrrolo[2,3-d]pyrimidin-5-yl)-2-aminoethyl] benzoyl]-L-glutamate

To a solution of the compound of Example 15 (260 mg) in ethanol (30 ml) was added 10% Pd-C (200 mg), followed by stirring at room temperature for 14 hours under an atmosphere of hydrogen. The catalyst was removed by filtration with celite, and the solvent was distilled off under reduced pressure to give the title compound (80 mg). This compound was used in the next reaction without purification.

### Example 18

Production of N-[4-[1-(2-amino-4-hydroxy-7H-pyrrolo[2,3-d]pyrimidin-5-yl)-2-aminoethyl]benzoyl]-L-glutamic acid

The procedure of Example 2 was followed starting with the compound of Example 17 (75 mg) to give the title compound (41 mg).
¹H-NMR (Me₂SO-d₆ + D₂O) δ: 1.83-2.03 (2H,m), 2.04-2.12 (2H,m), 3.38-3.72 (2H,m), 4.19-4.23 (1H,m), 4.61 (1H,m), 6.59 (1H,s), 7.43 (2H,d,J=8 Hz), 7.73 (2H,d,J=8 Hz)

### Example 19

Production of diethyl N-[4-[1,1-dimethyl(2-amino-4-hydroxy-7H-pyrrolo[2,3-d]pyrimidin-5-yl)methyl]benzoyl]-L-glutamate

The procedure of Example 7 was followed starting with methyl 4-(1,1-dimethyl(2-amino-4-hydroxy-7H-pyrrolo[2,3-d]pyrimidin-5-yl)methyl]benzoate (2.82 g) to give the title compound (3.77 g).
¹H-NMR (Me₂SO-d₆) δ: 1.16(3H,t,J=7.0 Hz), 1.18(3H,t,J=7.0 Hz), 1.69(6H,s), 1.87-2.20(2H,m), 2.42(2H,t,J=7.4 Hz), 4.04(2H,q,J=7.0 Hz), 4.10(2H,q,J=7.0 Hz), 4.85-4.98(1H,m), 5.99(2H,s), 6.31(1H,d,J=2.0 Hz), 7.33(2H,d,J=8.4 Hz), 7.69(2H,d,J=8.4 Hz), 8.58(1H,d,J=7.6 Hz), 9.85(1H,s), 10.72(1H,d,J=2.0 Hz)
IR(KBr): 3350, 2980, 2930, 1730, 1670, 1630, 1610, 1580, 1530 cm⁻¹

### Example 20

Production of N-[4-[1,1-dimethyl(2-amino-4-hydroxy-7H-pyrrolo[2,3-d]pyrimidin-5-yl)methyl]benzoyl]-L-glutamic acid

The procedure of Example 2 was followed starting with the compound of Example 19 (1.70 g) to give the title compound (1.32 g).
¹H-NMR (Me₂SO-d₆) δ: 1.69(6H,s), 1.86-2.17(2H,m), 2.35(2H,t,J=7.2 Hz), 4.32-4.46(1H,m), 5.99(2H,s), 6.30(1H,d,J=2.0 Hz), 7.32(2H,d,J=8.4 Hz), 7.70(2H,d,J=8.4 Hz), 8.46(1H,d,J=7.6 Hz), 9.86(1H,s), 10.72(1H,d,J=2.0 Hz)
IR(KBr): 3350, 2970, 1690, 1645, 1610 cm⁻¹

### Example 21

Production of methyl [N-[4-[1-(2-amino-4-hydroxy-7H-pyrrolo[2,3-d]pyrimidin-5-yl)propyl]benzoyl]-O¹-methyl-γ-L-glutamyl]-γ-benzyl-L-glutamate

The procedure of Example 1 was followed starting with t-butyl 4-[1-(2-amino-4-hydroxy-7H-pyrrolo[2,3-d]pyrimidin-5-yl)propyl]benzoate (316 mg), using [O¹-methyl-γ-L-glutamyl]-γ-benzyl-L-glutamic acid methyl trifluoroacetate (496 mg) instead of diethyl glutamate hydrochloride to give the title compound (535 mg).
¹H-NMR (Me₂SO-d₆) δ: 0.80(3H,t,J=7.0 Hz), 1.76-2.23(6H,m), 2.27(2H,t,J=8.0 Hz), 2.43(2H,t,J=8.0 Hz), 3.59(3H,s), 3.63(3H,s), 4.10-4.49(3H,m), 5.08(2H,s), 5.99(2H,s), 6.41(1H,s), 7.35(5H,s), 7.40(2H,d,J=8.4 Hz), 7.75(2H,d,J=8.4 Hz), 8.28(1H,d,J=8.0 Hz), 8.63(1H,d,J=8.0 Hz), 10.07(1H,s), 10.74(1H,s)
IR(KBr): 3300, 2950, 1740, 1680-1620, 1530 cm⁻¹

### Example 22

Production of [N-[4-[1-(2-amino-4-hydroxy-7H-pyrrolo[2,3-d]pyrimidin-5-yl)propyl]benzoyl]-γ-L-glutamyl]-L-glutamic acid

The procedure of Example 2 was followed starting with the compound of Example 21 (512 mg), using 3.34 ml of IN aqueous sodium hydroxide solution to give the title compound (292 mg).
¹H-NMR (Me₂SO-d₆) δ: 0.81(3H,t,J=7.0 Hz), 1.63-2.38(10H,m), 4.09-4.49(3H,m), 5.99(2H,s), 6.40(1H,s), 7.41(2H,d,J=8.0 Hz), 7.77(2H,d,J=8.0 Hz), 8.12(1H,d,J=7.6 Hz), 8.54(1H,d,J=7.6 Hz), 10.08(1H,s), 10.73(1H,s)
IR(KBr): 3350, 2960, 2930, 1700, 1640, 1540 cm⁻¹

### Pharmaceutical Example 1

The compound obtained in Example 4 (50 mg/tablet), lactose (250 mg/tablet), corn starch (51 mg/tablet) and hydroxypropylcellulose L (9 mg/tablet) were blended and granulated in the conventional manner and after addition of corn starch (8 mg/tablet) and magnesium stearate (2 mg/tablet), the granules were compression-molded to give tablets (370 mg/tablet).

### Pharmaceutical Example 2

In one liter of physiological saline was dissolved 10 g of the sodium salt of the compound of Example 4 and the solution was filtered through a micropore filter and filled in 2.2 ml portions into ampules, which were then sealed. These ampules were sterilized at 110°C for 30 minutes to provide ampules for subcutaneous, intravenous or intramuscular injection.

### Pharmaceutical Example 3

In one liter of distilled water were dissolved 5 g of the hydrochloride of the compound obtained in Example 4 and 10 g of mannitol and the solution was filtered through a bacterial filter and filled in 2 ml portions into ampules. The ampules were dried in a freeze-dryer and sealed to provide ampules for extemporaneous reconstitution. For use, the ampule is unsealed and the content is dissolved in 2 ml of physiological saline or the like for injection.

### Effect of the Invention

There is provided a novel thymidylate synthetase inhibitor having highly selective toxicity to various tumor cells (particularly human lung cancer cells) and exhibiting high therapeutic efficacy even against methotrexate-resistant tumor cells.

## Claims

1. A compound of the general formula: 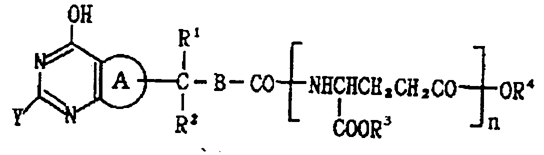 wherein a ring A represents a pyrrole or pyrroline ring which may be substituted; B means a divalent 5- or 6-membered homo- or hetero-cyclic group which may be substituted; Y means a hydrogen atom, a halogen atom, or a group bonded through a carbon, nitrogen or sulfur atom; R¹ and R² are the same or different and each means a hydrogen atom or a lower hydrocarbon group which may be substituted; -COOR³ and -COOR⁴ are the same or different and each means a carboxyl group which may be esterified; n means a whole number of 1 through 6; R³ may be different over n occurrences, or a salt thereof.

2. A compound of the general formula: 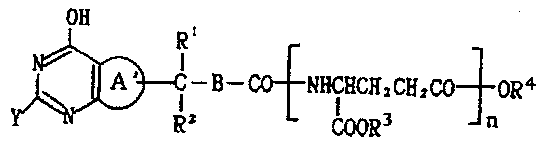 wherein a ring A' represents an N-substituted pyrrole or pyrroline ring; B means a divalent 5- or 6-membered homo- or hetero-cyclic group which may be substituted; Y means a hydrogen atom, a halogen atom, or a group bonded through a carbon, nitrogen or sulfur atom; R¹ and R² are the same or different and each means a hydrogen atom or a lower hydrocarbon group which may be substituted; -COOR³ and -COOR⁴ are the same or different and each means a carboxyl group which may be esterified; n means a whole number of 1 through 6; R³ may be different over n occurrences, or a salt thereof.

3. A compound as claimed in claim 1, wherein A is a pyrrole or pyrroline ring which may be substituted with one or two substituents selected from the group consisting of a C₁₋₄ alkyl, C₂₋₄ alkenyl, C₂₋₄ alkynyl, C₃₋₆ cycloalkyl, halogen, C₁₋₄ alkanoyl, benzoyl, halobenzoyl, mono-, di- or tri-C₁₋₄ alkoxybenzoyl, cyano, carboxy, carbamoyl, nitro, hydroxy, hydroxy-C₁₋₄ alkyl, C₁₋₄ alkoxy-C₁₋₄ alkyl, C₁₋₄ alkoxy, mercapto, C₁₋₄ alkylthio, amino, mono- or di-C₁₋₄ alkylamino, C₁₋₄ alkanoylamino, phenyl, halophenyl, mono-, di- or tri-C₁₋₄ alkoxyphenyl, benzyl, halobenzyl and C₁₋₄ alkoxybenzyl group.

4. A compound as claimed in claim 2, wherein A' is an N-substituted pyrrole or pyrroline ring, the substituent being a C₁₋₄ alkyl, C₂₋₄ alkenyl, C₂₋₄ alkynyl, C₃₋₆ cycloalkyl, C₁₋₄ alkanoyl, benzoyl, halobenzoyl, mono-, di- or tri-C₁₋₄ alkoxybenzoyl, hydroxy-C₁₋₄ alkyl, C₁₋₄ alkoxy-C₁₋₄ alkyl, phenyl, halophenyl, mono-, di- or tri-C₁₋₄ alkoxyphenyl, benzyl, halobenzyl or C₁₋₄ alkoxybenzyl group.

5. A compound as claimed in claim 1, which is a compound of the formula: 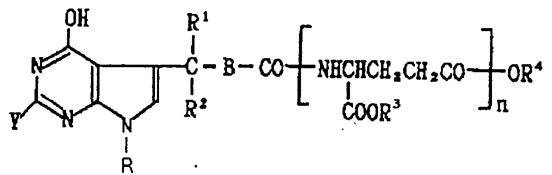 wherein R is a hydrogen atom or a C₁₋₄ alkyl, C₂₋₄ alkenyl, C₂₋₄ alkynyl, C₃₋₄ cycloalkyl, C₁₋₄ alkanoyl, benzoyl, halobenzoyl, mono-, di- or tri-C₁₋₄ alkoxybenzoyl, hydroxy-C₁₋₄ alkyl, C₁₋₄ alkoxy-C₁₋₄ alkyl, phenyl, halophenyl, mono-, di- or tri-C₁₋₄ alkoxyphenyl, benzyl, halobenzyl, C₁₋₄ alkoxybenzyl or diphenylmethyl group, and the other symbols have the same meanings as defined in claim 1.

6. A compound as claimed in claim 5, wherein R is a hydrogen atom.

7. A compound as claimed in claim 5, wherein R is a C₁₋₄ alkyl, C₂₋₄ alkenyl, C₂₋₄ alkynyl, C₁₋₄ alkanoyl, benzoyl, halobenzoyl, mono-, di- or tri-C₁₋₄ alkoxybenzoyl, hydroxy-C₁₋₄ alkyl, C₁₋₄ alkoxy-C₁₋₄ alkyl, benzyl, halobenzyl, C₁₋₄ alkoxybenzyl or diphenylmethyl group.

8. A compound as claimed in claim 1, wherein B is an unsubstituted or substituted divalent 5- or 6-membered cyclic hydrocarbon group or unsubstituted or substituted divalent 5- or 6-membered heterocyclic group containing 1 to 3 hetero-atoms selected from N, O and S as ring-constituent members, the substituent(s) being one or two selected from the group consisting of C₁₋₄ alkyl, C₂₋₄ alkenyl, C₂₋₄ alkynyl, C₃₋₆ cycloalkyl, halogen, hydroxy, C₁₋₄ alkoxy, di-C₁₋₄ alkylamino, halo-C₁₋₄ alkyl, oxo, C₁₋₄ acyl and C₁₋₄ alkoxy-C₁₋₄ alkyl.

9. A compound as claimed in claim 1, wherein B is an unsubstituted or substituted, 1,3- or 3,5-cyclopentadien-1,3-ylene, cyclopenten-(1,3-, 1,4- or 3,5-)ylene, cyclopentan-1,3-ylene, thiophen-(2,4-, 2,5- or 3,4-)ylene, furan-(2,4-, 2,5- or 3,4-)ylene, pyrrol-(1,3-, 2,4-, 2,5- or 3,4-)ylene, thiazol-(2,4- or 2,5-)ylene, imidazol-(1,4-, 2,4- or 2,5-)ylene, thiadiazol-2,5-ylene, phenyl-(1,3- or 1,4-)ylene, cyclohexan-(1,3- or 1,4-)ylene, cyclohexen-(1,3-, 1,4-, 1,5-, 3,5- or 3,6-)ylene, 1,3-cyclohexadien-(1,3-, 1,4-, 1,5-, 2,4-, 2,5- or 2,6-)ylene, 1,4-cyclohexadien-(1,3-, 1,4- or 1,5-)ylene, pyridin-(2,4-, 2,5-, 2,6- or 3,5-)ylene, pyran-(2,4-, 2,5-, 2,6-, 3,5-, 3,6- or 4,6-)ylene, pyrazin-(2,5- or 2,6-)ylene, pyrimidin-(3,4- or 2,5-)ylene ), pyridazin-3,5-ylene or partially or completely reduced form thereof, the substituent(s) being one or two selected from the group consisting of C₁₋₄ alkyl, C₂₋₄ alkenyl, C₂₋₄ alkynyl, C₃₋₆ cycloalkyl, halogen, hydroxy, C₁₋₄ alkoxy, di-C₁₋₄ alkylamino, halo-C₁₋₄ alkyl, oxo, C₁₋₄ acyl and C₁₋₄ alkoxy-C₁₋₄ alkyl.

10. A compound as claimed in claim 1, wherein B is an unsubstituted or substituted phenyl-1,4-ylene, thiophen-2,5-ylene, thiazol-2,5-ylene or pyridin-2,5-ylene, the substituent(s) being one or two selected from the group consisting of C₁₋₄ alkyl, C₂₋₄ alkenyl, C₂₋₄ alkyny, C₃₋₆ cycloalkyl, halogen, hydroxy, C₁₋₄ alkoxy, di-C₁₋₄ alkylamino, halo-C₁₋₄ alkyl, oxo, C₁₋₄ acyl and C₁₋₄ alkoxy-C₁₋₄ alkyl.

11. A compound as claimed in claim 1, wherein Y is
i) a hydrogen atom,
ii) a halogen,
iii) cyano, carboxy, carbamoyl, amino, nitro, hydroxy or mercapto,
iv) a C₁₋₄ alkyl, C₂₋₄ alkenyl, C₂₋₄ alkynyl or C₃₋₆ cycloalkyl which may be substituted with one or two substituents selected from the group consisting of a C₁₋₄ alkyl, C₂₋₄ alkenyl, C₂₋₄ alkynyl, C₃₋₈ cycloalkyl, halogen, hydroxy, oxo, C₁₋₄ alkoxy, di-C₁₋₄ alkylamino, halo-C₁₋₄ alkyl, C₁₋₄ acyl, hydroxy-C₁₋₄ alkyl and C₁₋₄ alkoxy-C₁₋₄ alkyl,
v) a C₆₋₁₀ aryl which may be substituted with one or two substituents selected from the group consisting of a C₁₋₄ alkyl, C₂₋₄ alkenyl, C₂₋₄ alkynyl, C₃₋₈ cycloalkyl, halogen, hydroxy, oxo, C₁₋₄ alkoxy, di-C₁₋₄ alkylamino, halo-C₁₋₄ alkyl, C₁₋₄ acyl, hydroxy-C₁₋₄ alkyl and C₁₋₄ alkoxy-C₁₋₄ alkyl,
vi) a 5- or 6-membered heterocyclic group containing 1 to 4 hetero-atoms selected from N, S and O which may be substituted with one or two substituents selected from the group consisting of a C₁₋₄ alkyl, C₂₋₄ alkenyl, C₂₋₄ alkynyl, C₃₋₈cycloalkyl, halogen, hydroxy, oxo, C₁₋₄ alkoxy, di-C₁₋₄ alkylamino, halo-C₁₋₄ alkyl, C₁₋₄ acyl, hydroxy-C₁₋₄ alkyl and C₁₋₄ alkoxy-C₁₋₄ alkyl,
vii) an C₁₋₄ alkoxy, C₁₋₄ alkylthio, C₁₋₄ alkylcarbonylamino or C₁₋₄ alkylcarbonyloxy wherein the alkyl moiety may be substituted with one or two substituents selected from the group consisting of a C₁₋₄ alkyl, C₂₋₄ alkenyl, C₂₋₄ alkynyl, C₃₋₈ cycloalkyl, halogen, hydroxy, oxo, C₁₋₄ alkoxy, di-C₁₋₄ alkylamino, halo-C₁₋₄ alkyl, C₁₋₄ acyl, hydroxy-C₁₋₄ alkyl and C₁₋₄ alkoxy-C₁₋₄ alkyl,
viii) an C₆₋₁₀ aryloxy, C₆₋₁₀ arylthio, C₆₋₁₀ aroylamino or C₆₋₁₀ aroyloxy wherein the aryl moiety may be substituted with one or two substituents selected from the group consisting of a C₁₋₄ alkyl, C₂₋₄ alkenyl, C₂₋₄ alkynyl, C₃₋₈ cycloalkyl, halogen, hydroxy, oxo, C₁₋₄ alkoxy, di-C₁₋₄ alkylamino, halo-C₁₋₄ alkyl, C₁₋₄ acyl, hydroxy-C₁₋₄ alkyl and C₁₋₄ alkoxy-C₁₋₄ alkyl,
ix) a heterocyclyloxy, heterocyclylthio, heterocyclylcarbonylamino or heterocyclylcarbonyloxy wherein the heterocycle is a 5- or 6-membered heterocyclic group containing 1 to 4 hetero-atoms selected from N, S and O which may be substituted with one or two substituents selected from the group consisting of a C₁₋₄ alkyl, C₂₋₄ alkenyl, C₂₋₄ alkynyl, C₃₋₈ cycloalkyl, halogen, hydroxy, oxo, C₁₋₄ alkoxy, di-C₁₋₄ alkylamino, halo-C₁₋₄ alkyl, C₁₋₄ acyl, hydroxy-C₁₋₄ alkyl and C₁₋₄ alkoxy-C₁₋₄ alkyl, or
x) a mono- or di-substituted Amino group, the substituent(s) being selected from the above iv), v) and vi).

12. A compound as claimed in claim 1, wherein Y is an amino group.

13. A compound as claimed in claim 1, wherein R¹ and R² are, the same or different, a hydrogen atom or a C₁₋₄ alkyl, C₂₋₄ alkenyl, C₂₋₄ alkynyl or C₃₋₆ cycloalkyl which may be substituted with one or two substituents selected from the group consisting of C₁₋₄ alkyl, C₂₋₄ alkenyl, C₂₋₄ alkynyl, C₃₋₈ cycloalkyl, halogen, hydroxy, oxo, C₁₋₄ alkoxy, di-C₁₋₄ alkylamino, halo-C₁₋₄ alkyl, C₁₋₄ acyl, hydroxy-C₁₋₄ alkyl, C₁₋₄ alkoxy-C₁₋₄ alkyl, amino and nitro.

14. A compound as claimed in claim 1, wherein R¹ and R² are, the same or different, a hydrogen atom or a C₁₋₄ alkyl, C₂₋₄ alkenyl, C₂₋₄ alkynyl or C₃₋₆ cycloalkyl which may be substituted with one or two substituents selected from the group consisting of C₁₋₄ alkyl, C₂₋₄ alkenyl, C₂₋₄ alkynyl, C₃₋₈ cycloalkyl, halogen, hydroxy, oxo, C₁₋₄ alkoxy, di-C₁₋₄ alkylamino, halo-C₁₋₄ alkyl, C₁₋₄ acyl, hydroxy-C₁₋₄ alkyl and C₁₋₄ alkoxy-C₁₋₄ alkyl.

15. A compound as claimed in claim 1, wherein R³ and R⁴ are, the same or different,
i) a hydrogen atom,
ii) a C₁₋₅ alkyl,
iii) a benzyl which may be substituted with one to three substituents selected from the group consisting of nitro and a C₁₋₄ alkoxy group, or
iv) a phenyl which may be substituted with one to three substituents selected from the group consisting of nitro and a C₁₋₄ alkoxy group.

16. A compound as claimed in claim 1, wherein n is 1.

17. A compound as claimed in claim 1, wherein the salt is a pharmaceutically acceptable salt.

18. A compound as claimed 5, wherein
Y is an amino group,
R is a hydrogen atom or C₁₋₄ alkyl,
R ¹ and R² are, the same or different, a hydrogen atom or a C₁₋₄ alkyl, C₂₋₄ alkenyl or C₂₋₄ alkynyl which may be substituted with an amino or nitro,
B is a phenyl-1,4-ylene or thiophen-2,5-ylene which may be substituted with a halogen,
R³ and R⁴ are a hydrogen atom or a C₁₋₅ alkyl, and
n is 1 or 2.

19. A compound as claimed in claim 5, wherein
Y is an amino group,
R is a hydrogen atom or C₁₋₄ alkyl,
R¹ and R² are, the same or different, a hydrogen atom or a C₁₋₄ alkyl or C₂₋₄ alkenyl which may be substituted with an amino or nitro,
B is a phenyl-1,4-ylene or thiophen-2,5-ylene which may be substituted with a halogen,
R³ and R⁴ are a hydrogen atom or a C₁₋₅ alkyl, and
n is 1 or 2.

20. A compound as claimed in claim 5, wherein
Y is an amino group,
R is a hydrogen atom,
R¹ and R² are, the same or different, a hydrogen atom or a C₁₋₄ alkyl, C₂₋₄ alkenyl or C₂₋₄ alkynyl,
B is a phenyl-1,4-ylene or thiophen-2,5-ylene which may be substituted with a halogen,
R³ and R⁴ are a hydrogen atom, and
n is 1 or 2.

21. A compound as claimed in claim 5, wherein
Y is an amino group,
R is a hydrogen atom,
R¹ and R² are, the same or different, a hydrogen atom or a C₁₋₄ alkyl or C₂₋₄ alkenyl,
B is a phenyl-1,4-ylene or thiophen-2,5-ylene,
R³ and R⁴ are a hydrogen atom, and
n is 1 or 2.

22. A compound as claimed in claim 5, wherein
Y is an amino group,
R is a hydrogen atom or C₁₋₄ alkyl,
R¹ and R² are, the same or different, a hydrogen atom or a C₁₋₄ alkyl or C₂₋₄ alkenyl which may be substituted with an amino or nitro,
B is a phenyl-1,4-ylene or thiophen-2,5-ylene which may be substituted with a halogen,
R³ and R⁴ are a hydrogen atom, and
n is 1.

23. A compound as claimed in claim 5, wherein B is a phenyl-1,4-ylene.

24. A compound as claimed in claim 5, wherein B is a thiophen-2,5-ylene.

25. A compound as claimed in claim 5, wherein R¹ and R² are a hydrogen atom.

26. A compound as claimed in claim 5, wherein R³ and R⁴ are a hydrogen atom.

27. A compound as claimed in claim 5, wherein R is a C₁₋₃ alkyl group.

28. A compound as claimed in claim 27, wherein the C₁₋₃ alkyl group is methyl.

29. A compound as claimed in claim 1, which is N-[5-(2-amino-4-hydroxy-7H-pyrrolo[2,3-d]pyrimidin-5-yl )methyl-2-thenoyl]-L-glutamic acid.

30. A compound as claimed in claim 1, which is N-[4-[1-(2-amino-4-hydroxy-7H-pyrrolo[2,3-d]pyrimidin-5-yl)ethyl]benzoyl]-L-glutamic acid.

31. A compound as claimed in claim 1, which is N-[4-[1-(2-amino-4-hydroxy-7H-pyrrolo[2,3-d]pyrimidin-5-yl)propyl]benzoyl]-L-glutamic acid.

32. A compound as claimed in claim 1, which is N-[4-[1-(2-amino-4-hydroxy-7H-pyrrolo[2,3-d]pyrimidin-5-yl)-2-propenyl]benzoyl]-L-glutamic acid.

33. A compound as claimed in claim 1, which is N-[4-(2-amino-4-hydroxy-7H-pyrrolo[2,3-d]pyrimidin-5-yl)methyl-2-fluorobenzoyl]-L-glutamic acid.

34. A compound as claimed in claim 1, which is N-[4-[1-(2-amino-4-hydroxy-7H-pyrrolo[2,3-d]pyrimidin-5-yl)-3-butenyl]benzoyl]-L-glutamic acid.

35. A compound as claimed in claim 1, which is N-[4-[1-(2-amino-4-hydroxy-7H-pyrrolo[2,3-d]pyrimidin-5-yl)-3-butynyl]benzoyl]-L-glutamic acid.

36. A compound as claimed in claim 1, which is N-[4-[1,1-dimethyl(2-amino-4-hydroxy-7H-pyrrolo[2,3-d]pyrimidin-5-yl)methyl]benzoyl]-L-glutamic acid.

37. A compound as claimed in claim 1, which is N-[4-[1,1-diethyl(2-amino-4-hydroxy-7H-pyrrolo[2,3-d]pyrimidin-5-yl)methyl]benzoyl]-L-glutamic acid.

38. A process for producing a compound of claim 1, which comprises reacting a compound of the-general formula: 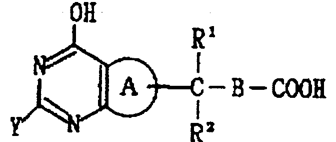 wherein the symbols have the same meanings as defined in claim 1, or a reactive derivative of the carboxyl group thereof with a compound of the general formula: 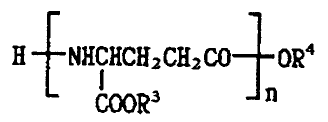 wherein the symbols have the same meanings as defined in claim 1.

39. A thymidylate synthase inhibitor composition which contains an effective amount of a compound of the general formula: 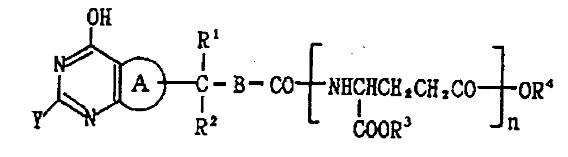 wherein a ring A represents a pyrrole or pyrroline ring which may be substituted; B means a divalent 5- or 6- membered homo- or hetero-cyclic group which may be substituted; Y means a hydrogen atom, a halogen atom, or a group bonded through a carbon, nitrogen or sulfur atom; R¹ and R² are the same or different and each means a hydrogen atom or a lower hydrocarbon group which may be substituted; -COOR³ and -COOR⁴ are the same or different and each means a carboxyl group which may be esterified; n means a whole number of 1 through 6; R³ may be different over n occurrences, or a pharmaceutically acceptable salt thereof together with one or more carriers, diluents or excipients.

40. Method of inhibiting a thymidylate synthase in a mammal in need thereof which comprises administrating to such mammal of an effective amount of a compound of the general formula: 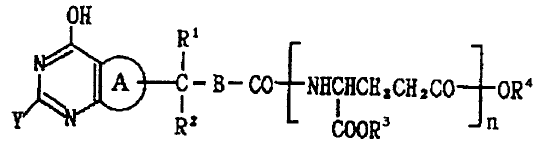 wherein a ring A represents a pyrrole or pyrroline ring which may be substituted; B means a divalent 5- or 6- membered homo- or hetero-cyclic group which may be substituted; Y means a hydrogen atom, a halogen atom, or a group bonded through a carbon, nitrogen or sulfur atom; R¹ and R² are the same or different and each means a hydrogen atom or a lower hydrocarbon group which may be substituted; -COOR³ and -COOR⁴ are the same or different and each means a carboxyl group which may be esterified; n means a whole number of 1 through 6; R³ may be different over n occurrences, or a pharmaceutically acceptable salt thereof.

41. A compound of the general formula: 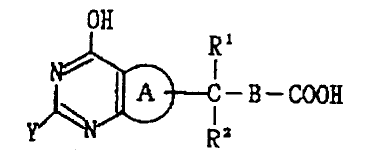 wherein the symbols have the same meanings as defined in claim 1, or a salt or ester thereof.

42. A compound of the general formula: 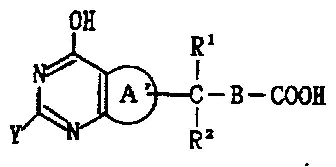 wherein a ring A' represents an N-substituted pyrrole or pyrroline ring; B means a divalent 5- or 6-membered homo- or hetero-cyclic group which may be substituted; Y means a hydrogen atom, a halogen atom or a group bonded through a carbon, nitrogen or sulfur atom; R¹ and R² are the same or different and each means a hydrogen atom or a lower hydrocarbon group which may be substituted, or a salt or ester thereof.

43. Use of a compound of the general formula: 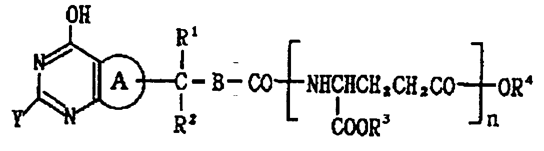 wherein a ring A represents a pyrrole or pyrroline ring which may be substituted; B means a divalent 5- or 6- membered homo- or hetero-cyclic group which may be substituted; Y means a hydrogen atom, a halogen atom, or a group bonded through a carbon, nitrogen or sulfur atom; R¹ and R² are the same or different and each means a hydrogen atom or a lower hydrocarbon group which may be substituted; -COOR³ and -COOR⁴ are the same or different and each means a carboxyl group which may be esterified; n means a whole number of 1 through 6; R³ may be different over n occurrences, or a pharmaceutically acceptable salt thereof in the preparation of a thymidylate synthase inhibitor.
